# EUROPEAN PATENT SPECIFICATION

(11) **EP 2 293 847 B1**
(45) Date of publication and mention of the grant of the patent: **25.03.2015**
(21) Application number: 09750137.3
(22) Date of filing: 22.05.2009
(51) Int. Cl.: C07K 5/06, C07K 5/062, C07K 5/078, A61P 35/00, A61P 37/00, A61P 3/10, A61P 9/02, A61P 19/10, A61P 17/00, A61P 17/10

(54) **DEPSIPEPTIDES AND THEIR THERAPEUTIC USE**
DEPSIPEPTIDE UND IHRE THERAPEUTISCHE VERWENDUNG
DEPSIPEPTIDES ET LEUR UTILISATION THÉRAPEUTIQUE

(30) Priority: 22.05.2008 GB 0809329
(43) Date of publication of application: 16.03.2011
(73) Proprietor: Karus Therapeutics Limited, Abingdon, Oxfordshire OX14 4RZ (GB)
(72) Inventor: SHUTTLEWORTH, Stephen Joseph, Southampton Hampshire SO16 7NP (GB); SILVA, Franck Alexandre, Southampton Hampshire SO16 7NP (GB); TOMASSI, Cyrille Davy, Southampton Hampshire SO16 7NP (GB); CECIL, Alexander Richard Liam, Southampton Hampshire SO16 7NP (GB); HILL, Thomas James, Southampton Hampshire SO16 7NP (GB)
(74) Representative: Stevens, Fiona
(86) International application number: PCT/GB2009/050555
(87) International publication number: WO 2009/141659

(56) References cited:
- WO-A-2006/129105
- YUREK-GEORGE ALEXANDER ET AL: "The first biologically active synthetic analogues of FK228, the depsipeptide histone deacetylase inhibitor" JOURNAL OF MEDICINAL CHEMISTRY, AMERICAN CHEMICAL SOCIETY, WASHINGTON, US, vol. 50, no. 23, 15 November 2007 (2007-11-15), pages 5720-5726, XP002470763 ISSN: 0022-2623
- TANABE ET AL: "Inhibition of fungal ABC transporters by unnarmicin A and unnarmicin C, novel cyclic peptides from marine bacterium" BIOCHEMICAL AND BIOPHYSICAL RESEARCH COMMUNICATIONS, ACADEMIC PRESS INC. ORLANDO, FL, US, vol. 364, no. 4, 29 October 2007 (2007-10-29), pages 990-995, XP022344223 ISSN: 0006-291X

## Description

### Field of the Invention

The present invention relates to depsipeptides which act as inhibitors of histone deacetylase (HDAC) and therefore have therapeutic utility

### Background of the Invention

HDACs are zinc metalloenzymes that catalyse the hydrolysis of acetylated lysine residues. In histones, this returns lysines to their protonated state and is a global mechanism of eukaryotic transcriptional control, resulting in tight packaging of DNA in the nucleosome. Additionally, reversible lysine acetylation is an important regulatory process for non-histone proteins. Thus, compounds that are able to modulate HDAC have important therapeutic potential.

The natural products FK228 (Structure I) and Spiruchostatin A (Structure II) are depsipeptides that have been reported to have potential as HDAC inhibitors. The term depsipeptide describes a class of oligopeptides or polypeptides that have both ester and peptide links the chain.

FK228 is a cyclic depsipeptide containing 4 monomer units together with a cross-ring bridge. This compound, under the trade name of Romidepsin®, has been tested as a therapeutic in human trials and shown that it has valuable effects on a number of diseases.

Spiruchostatin A is a cyclic depsipeptide that is structurally related to FK228: it is a cyclic depsipeptide containing a tri-peptide, a statine unit and a cross-ring bridge.

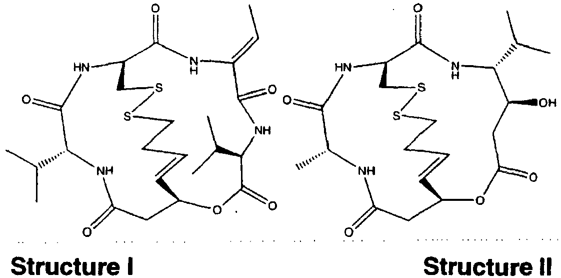

However, because both FK228 and Spiruchostatin A are natural products, they are not amenable to optimization for use as a therapeutic agent.

Analogues of these compounds are disclosed in WO2006/129105 and further such compounds are disclosed in PCT/GB2007/050709 and in Yurek-George A et al, J Med chem, 2007, 50, 5720-5726. They may have improved HDAC inhibitory properties with respect to FK228 and Spiruchostatin A or other drug-like properties that make them more useful as medicines. These compounds have the general structures shown in Structures IA & IIA wherein by R¹, R², R³ & R⁴ are the same or different and represent hydrogen or an amino acid side chain moiety (from either a natural or an unnatural amino acid) and each R⁵ is the same or different and represents hydrogen or C₁-C₆ alkyl, C₂-C₆ alkenyl or C₂-C₆ alkynyl, and Pr is hydrogen or an alcohol protecting group.

WO2008/062201 discloses depsipeptides of structures III & IV, with slight modifications in the 4-thio-butyl-1-ene metallophile.

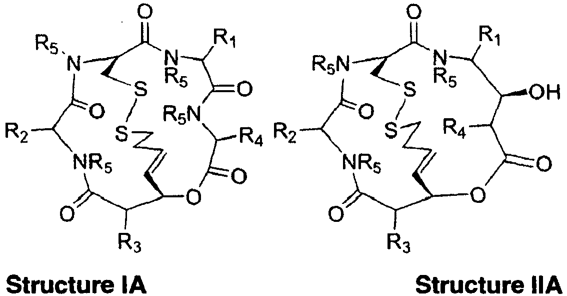

Without being constrained by theory, it is believed that Structures V and VI are formed inside the cell from Structures I & II respectively, by reduction of the disulfide bond, and that the 4-thio-butyl-1-ene so formed is a critical part of the mechanism of action of the compound, forming a metallophile capable of binding Zinc in the active site of HDAC.

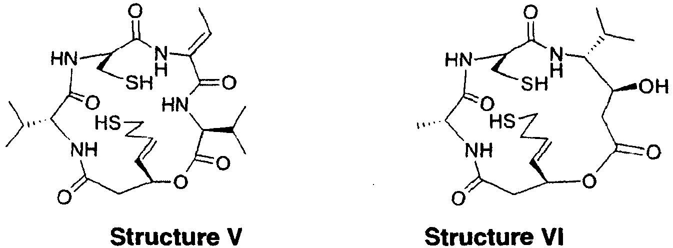

This concept is supported by the observation that FR-901375, a cyclic depsipeptide HDAC inhibitor with quite a different ring structure, has the same disulfide-containing bridge across the ring as is seen in FK228 and Spiruchostatin A.

### Summary of the Invention

The present invention provides compounds of Structures III & IV, which are derivatives of Structures V & VI, and of structures related to them in ways that will be apparent to those skilled in the art, in which the 4-thio-butyl-1-ene metallophile is replaced by an alternative metallophile and which, surprisingly, are found to be effective inhibitors of HDAC enzymes, and have properties which indicate that they may have greater potential as treatments for human disease. These compounds are hereinafter designated members of the class of compounds called Alternative Metallophile Depsipeptides (AMDs), and are defined by Structure III or IV, as shown below. 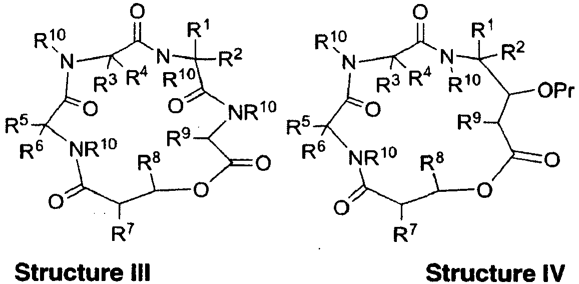 or a pharmaceutically acceptable salt thereof,
wherein:
R₁, R₂, R₃, R₄, R₅, R₆, R₇, and R₉ are the same or different and represent hydrogen or natural or unnatural amino acid side-chain moiety or R₁ and R₂, R₃ and R₄, or R₅ and R₆, taken together with the carbon atom to which they are attached, from a cyclic moiety containing between 3 and 8 carbon atoms;
each R₁₀ is the same of different and represents hydrogen or C₁-C₆ alkyl, C₂-C₆ alkenyl or C₂-C₆ alkynyl;
Pr is hydrogen or an alcohol protecting group;
R₈ represents -CR₁₁R₁₁-CR₁₁R₁₁-(CH₂)ₙ-R₁₂, CR₁₁=CR₁₁-(CH₂)ₙ-R₁₂, or -C≡C-(CH₂)ₙ-R₁₂;
R₁₁ is hydrogen, C₁-C₆ alkyl, aryl, C₂-C₆ alkenyl, C₂-C₆ alkynyl or heteroaryl;
R₁₂ is a linear or cyclic metal-chelating moiety, capable of binding with zinc in the active site of histone deacetylase, and is represented by a structure shown below: 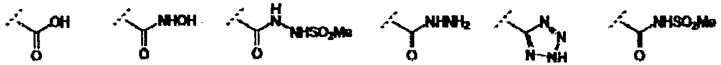 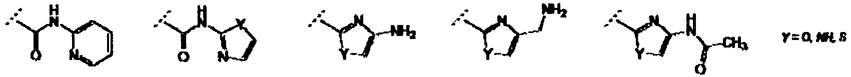 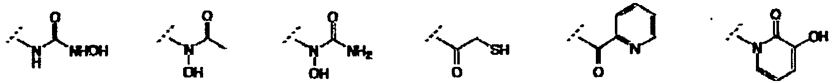 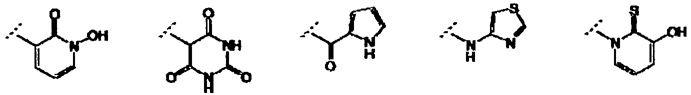 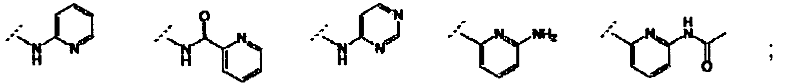 and
   n is an integer from 0 to 7,
   each unnatural amino acid side-chain moiety is independently selected from -(CH₂)₂-C(O)-O-C(CH₃)₃ (glutamic acid *t*-butyl ester), -(CH₂)₄-NH-C(O)-O-C(CH₃)₃ (N_{ε}-(*tert*-butoxycarbonyl)-lysine), -(CH₂)₃-NH-C(O)NH₂ (citrulline), -CH₂-CH₂OH (homoserine), -(CH₂)₂-CH₂NH₂ (ornithine), C₁-C₆ alkyl, C₂-C₆ alkenyl, C₂-C₆ alkynyl, aryl or a saturated or unsaturated heterocycle. -(CR₁₁R₁₁)ₓNR₁₁C(O)NR₁₁R₁₁, -(CR₁₁R₁₁)ₓ-NRC(O)NR₁₁R₁₃, -(CR₁₁R₁₁)ₓ-NR₁₁C(O)OR₁₄, -(CR₁₁R₁₁)ₓNR₁₁C(O)R₉, -(CR₁₁R₁₁)ₓNR₁₁C(O)R₁₃, -(CR₁₁R₁₁)ₓNR₁₁SO₂NR₁₁R₁₁, -(CR₁₁R₁₁)ₓ-NR₁₁SO₂NR₁₁R₁₃, -(CR₁₁R₁₁)ₓNR₁₁SO₃R₁₄, -(CR₁₁R₁₁)ₓ-NR₁₁SO₂R₁₄, -(CR₁₁R₁₁)ₓNR₁₁SO₂R₁₃, -(CR₁₁R₁₁)ₓ-C(O)NR₁₁R₁₁, -(CR₁₁R₁₁)ₓ-C(O)NR₁₁R₁₃, -(CR₁₁R₁₁)ₓ-CO₂R₁₁, -(CR₁₁R₁₁)ₓC(O)R₁₃, -(CR₁₁R₁₁)ₓSO₂NR₁₁R₁₁, -(CR₁₁R₁₁)ₓ-SO₂NR₁₁R₁₃, -(CR₁₁R₁₁)ₓ-SO₂R₁₃, -(CR₁₁R₁₁)ₓ-Ar;
   x is an integer from 1 to 10;
   R₁₃ is NR₁₁-C(O)R₁₄ or NR₁₁-SO₂R₁₄;
   R₁₄ is C₁-C₆ alkyl, aryl, C₂-C₆ alkenyl, C₂-C₆ alkynyl or heteroaryl; and
   Ar is functionalized and unfunctionalized phenyl, and functionalized and unfunctionalized 5- and 6-membered unsaturated aryl and heteroaryl rings.

The present invention further provides the use of an AMD as defined above or a pharmaceutically acceptable salt thereof, in the manufacture of a medicament for use as an inhibitor of HDAC.

### Description of the Invention

The invention is defined by the claims.

Synthesis of compounds of Structures III and IV is typically conducted using amino acids of which -C(O)-CR'R"-NH- forms part of the macrocycle and R' and R" are side-chain moieties. R₁, R₂, R₃, R₄, R₅, R₆ and R₉ (R₉ in Structure III only) may be introduced in this way. R₇ and R₉ (R₉ in Structure IV only) may be an amino acid side chain moiety but may not have been derived directly or indirectly from an amino acid as such.

As used herein, the term "amino acid side chain moiety" refers to any side chain that may be present in natural or unnatural amino acids. Examples of amino acid side chain moieties derived from unnatural amino acids, with the amino acids from which they are derived shown in brackets, are -(CH₂)₂-C(O)-O-C(CH₃)₃ (glutamic acid *t-*butyl ester), -(CH₂)₄-NH-C(O)-O-C(CH₃)₃ (N_{ε}-(*tert*-butoxycarbonyl)-lysine), -(CH₂)₃-NH-C(O)NH₂ (citrulline), -CH₂-CH₂OH (homoserine) and -(CH₂)₂-CH₂NH₂ (ornithine). Examples can also include alkyl, alkenyl, alkynyl, aryl, saturated and unsaturated heterocycles (functionalized and unfunctionalized).

The term 'amino-acid side chain moiety' can also include a number of unnatural amide and sulfonamide, aryl and heteroaryl side chains of the structure: -(CR₁₁R₁₁)ₓ-NR₁₁C(O)NR₁₁R₁₁, -(CR₁₁R₁₁)ₓ-NR₁₁C(O)NR₁₁R₁₃,-(CR₁₁R₁₁)ₓ-NR₁₁C(O)OR₁₄, -(CR₁₁R₁₁)ₓ-NR₁₁C(O)R₉, -(CR₁₁R₁₁)ₓ-NR₁₁C(O)R₁₃,-(CR₁₁R₁₁)ₓ-NR₁₁SO₂NR₁₁R₁₁, -(CR₁₁R₁₁)ₓ-NR₁₁SO₂NR₁₁R₁₃, -(CR₁₁R₁₁)ₓ-NR₁₁SO₃R₁₄, -(CR₁₁R₁₁)ₓ-NR₁₁SO₂R₁₄, -(CR₁₁R₁₁)ₓ-NR₁₁SO₂R₁₃, -(CR₁₁R₁₁)ₓ-C(O)NR₁₁R₁₁, -(CR₁₁R₁₁)ₓ-C(O)NR₁₁R₁₃, -(CR₁₁R₁₁)ₓ-CO₂R₁₁, -(CR₁₁R₁₁)ₓ-C(O)R₁₃, -(CR₁₁R₁₁)ₓ-SO₂NR₁₁R₁₁, -(CR₁₁R₁₁)ₓ-SO₂NR₁₁R₁₃, -(CR₁₁R₁₁)ₓ-SO₂R₁₃, -(CR₁₁R₁₁)ₓ-Ar, wherein x is an integer between 1 and 10; R₁₁ is hydrogen, alkyl, aryl, alkenyl, alkynyl, heteroaryl; R₁₃ is NR₁₁-C(O)R₁₄, NR₁₁-SO₂R₁₄; R₁₄ is alkyl, aryl, alkenyl, alkynyl, heteroaryl; and Ar is functionalized and unfunctionalized phenyl, and functionalized and unfunctionalized 5- and 6-membered unsaturated aryl and heteroaryl rings, including thiazole, tetrazole, imidazole, oxazole, isoxazole, thiophene, pyrazole and functionalized derivatives.

A C₁-C₆ alkyl group or moiety can be linear or branched. Typically, it is a C₁-C₄ alkyl group or moiety, for example methyl, ethyl, n-propyl, i-propyl, n-butyl, sec-butyl and t-butyl. Preferred examples include methyl, i-propyl and t-butyl.

A C₂-C₆ alkenyl group or moiety can be linear or branched. Typically, it is a C₂-C₄ alkenyl group or moiety. It is preferred that the alkenyl radicals are mono or di-unsaturated, more preferably monounsaturated. Examples include vinyl, allyl, 1-propenyl, isopropenyl, 1-butenyl, 2-butenyl and 3-butenyl.

A C₂-C₆ alkynyl group or moiety can be linear or branched. Typically, it is a C₂-C₄ alkynyl group or moiety.

The group 'Pr' represents hydrogen or a protecting group that forms an ether, an acetal or aminoacetal, an ester or a carbamic acid ester to protect a hydroxyl group. Preferably, Pr represents hydrogen or a protecting group selected from a benzyl group which is optionally substituted by C₁-C₆ alkoxy (for example methoxy), C₁-C₆ acyloxy (for example acetoxy), hydroxy and nitro, picolyl, picolyl-N-oxide, anthrylmethyl, diphenylmethyl, phenyl, t-butyl, adamantyl, C₁-C₆ acyloxymethyl (for example pivaloyloxymethyl, tertiary butoxycarbonyloxymethyl), C₁-C₆ alkoxymethyl (for example methoxymethyl, isobutoxymethyl), tetrahydropyranyl, benzylthiomethyl, phenylthiomethyl, acetamidomethyl, benzamidomethyl, tert-butyloxycarbonyl (BOC), acetyl and its derivatives, benzoyl and its derivatives, carbamoyl, phenylcarbamoyl and C₁-C₆ alkylcarbamoyl (for example methylcarbamoyl and ethylcarbamoyl). Most preferably, Pr is hydrogen.

The group R₈ (the 'alternative metallophile') must contain R₁₂, a metallophile capable of binding with Zinc in the active site of HDAC.

Preferably, R₈ is CR₁₁R₁₁-CR₁₁R₁₁-(CH₂)ₙ-R₁₂ wherein R₁₁ is as defined above. Examples of R₁₂ groups are shown below:

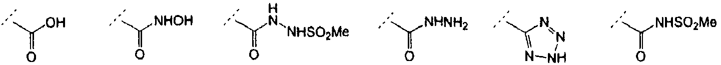

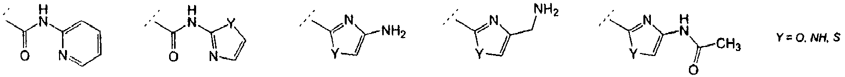

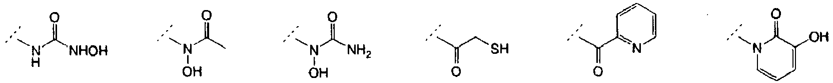

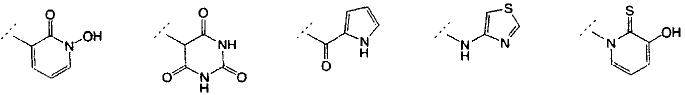

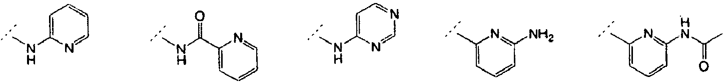

The following embodiments of the invention illustrate preferable R₁₂ groups:

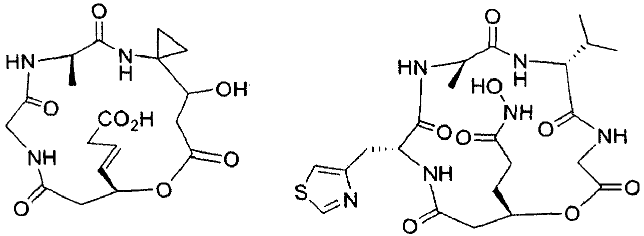

Preferably, the amino acid side chain moieties are those derived from natural amino acids. Examples of amino acid side chain moieties derived from natural amino acids, with the amino acids from which they are derived shown in brackets, are -H (Glycine), -CH₃ (Alanine), -CH(CH₃)₂ (Valine), -CH₂CH(CH₃)₂ (Leucine), -CH(CH₃)CH₂CH₃ (Isoleucine), -(CH₂)₄NH₂ (Lysine), -(CH₂)₃NHC(=NH)NH₂ (Arginine), -CH₂-(5-1*H*-imidazolyl) (Histidine), -CH₂CONH₂ (Asparagine), -CH₂CH₂CONH₂ (Glutamine), -CH₂COOH (Aspartic acid), -CH₂CH₂COOH (Glutamic acid), -CH₂-phenyl (Phenylalanine), -CH₂-(4-OH-phenyl) (Tyrosine), -CH₂-(3-1*H*-indolyl) (Tryptophan), -CH₂SH (Cysteine), -CH₂CH₂SCH₃ (Methioine), -CH₂OH (Serine), and -CH(OH)CH₃ (Threonine).

In one embodiment, each amino acid side chain is an amino acid side chain moiety present in a natural amino acid or is -(CH₂)₂-C(O)-O-C(CH₃)₃ (glutamic acid *t*-butyl ester), -(CH₂)₄-NH-C(O)-O-C(CH₃)₃ (N_{ε}-(*tert*butoxycarbonyl)-lysine), -(CH₂)₃-NH-C(O)NH₂ (citrulline), -CH₂-CH₂OH (homoserine) or -(CH₂)₂-CH₂NH₂ (ornithine).

In a further embodiment of the invention, one, two or all three pairs of side-chain moieties, (wherein R₁ and R₂ form one pair, R₃ and R₄ form another pair, and R₅ and R₆ form another pair), do not have hydrogen as an amino-acid side-chain moiety, and represent 2,2-bis-substituted alternative metallophile depsipeptides (AMDs).

In a further embodiment of the invention, one, two or all three pairs of side-chain moieties, (wherein R₁ and R₂ form one pair, R₃ and R₄ form another pair, and R₅ and R₆ form another pair), taken together with the carbon atom of the depsipeptide macrocycle to which they are attached, form cyclic moieties such that the carbon that is a part of the depsipeptide macrocycle is also part of a spirocyclic moiety, the external cyclic moiety being cycloalkyl, or other cyclic group which preferably has 3 to 8 atoms, e.g. cyclopropyl.

Preferred compounds of the invention are:

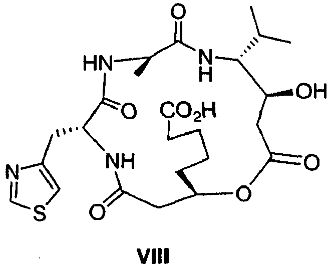

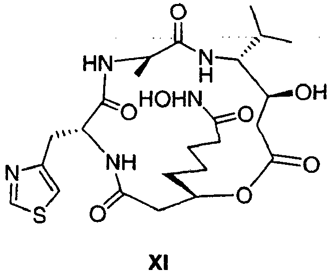

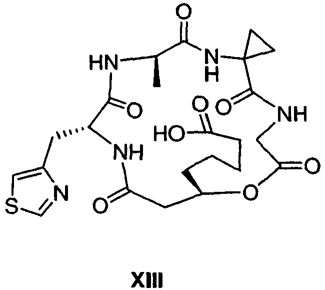

As used herein, a pharmaceutically acceptable salt is a salt with a pharmaceutically acceptable acid or base. Pharmaceutically acceptable acids include both inorganic acids such as hydrochloric, sulfuric, phosphoric, diphosphoric, hydrobromic or nitric acid and organic acids such as citric, fumaric, maleic, malic, ascorbic, succinic, tartaric, benzoic, acetic, methanesulfonic, ethanesulfonic, benzenesulfonic or p-toluenesulfonic acid. Pharmaceutically acceptable bases include alkali metal (e.g. sodium or potassium) and alkali earth metal (e.g. calcium or magnesium) hydroxides and organic bases such as alkyl amines, aralkyl amines or heterocyclic amines.

As used herein, the term "isostere" refers to a compound resulting from the exchange of an atom or a group of atoms with another, broadly similar, atom or group of atoms. In the compounds of Structures III or IV, the moieties which contain isosteric groups are preferably -NR₁₀-CR₁R₂-CO- (or -NR₁₀-CR₁R₂-CH(OPr)-), -NR₁₀-CHR₉-CO-O- (or -CHR₉-CO-O-), and -NR₁₀-CO-CR₃R₄-NR₁₀-CO-R₅R₆-NR₁₀-CO-CHR₇-. Examples of such isosteres are compounds of Structures III or IV wherein the moiety -NH- has been replaced by -CH₂-, -O- or -S-, the moiety -CO- has been replaced by -CS- or -C(=NH)- and the moiety -O- has been replaced by -S-, CH₂- or -NH-.

Said pharmaceutical composition typically contains up to 85 wt% of a compound of the invention. More typically, it contains up to 50 wt% of a compound of the invention. Preferred pharmaceutical compositions are sterile and pyrogen free. Further, the pharmaceutical compositions provided by the invention typically contain a compound of the invention which is a substantially pure optical isomer. Preferably, the pharmaceutical composition comprises a pharmaceutically acceptable salt of a compound of Structure III or IV.

The AMD may be prepared by conventional routes, for example using the following schemes wherein the functional groups are as defined above:

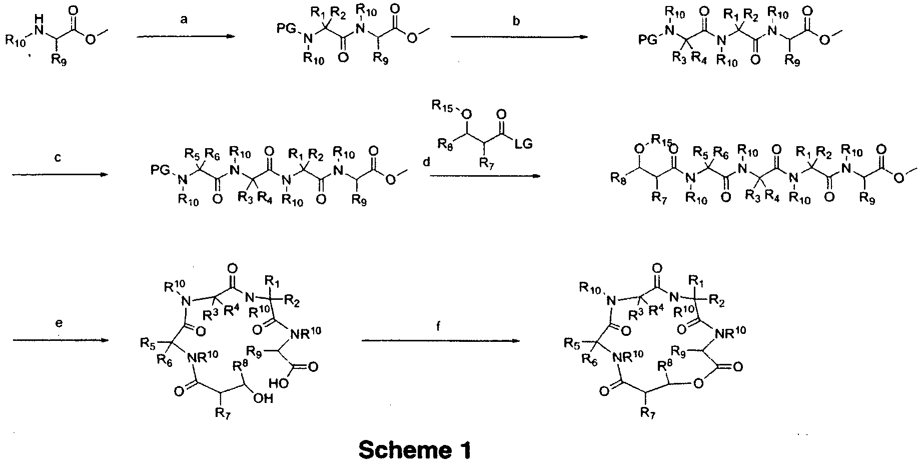

In Scheme 1 step (a), an amino acid ester bearing the side-chain R₉ is coupled with another, *N*-protected amino acid bearing the side chains R₁ and R₂ (where PG represents a conventional protecting group) to furnish the *N-*protected dipeptide ester. In step (b), the *N*-protecting group is removed, and the resulting dipeptide ester is coupled with an amino acid bearing the side chains R₃ and R₄. In step (c), the *N*-protecting group is removed, and the resulting tripeptide is coupled with an amino acid bearing the side chains R₅ and R₆ to liberate an *N*-protected tetrapeptide ester. In step (d), the *N*-protecting group is removed and the resulting tetrapeptide ester is coupled with a functionalised b-hydroxy acid derivative bearing the residues R₇ and R₈ the latter of which bears the Zinc-binding group in its terminus in protected or unprotected form; R₁₅ is a temporary blocking group which can be removed to produce a compound wherein R₁₅ is H; LG is a conventional leaving group, for example in the form of a chiral auxiliary as reported in Yurek-George, A. et al, *J. Am. Chem. Soc.* 2004, 126, 1030, or LG is an alcohol thus giving the free acid which allows for standard amide coupling. In step (e), the ester is hydrolysed, and cyclization is facilitated in step (f). Further chemical transformation of the terminal residue of R₈, for example ester hydrolysis to the corresponding carboxylic acid, a representative Zinc-binding group, may be conducted.

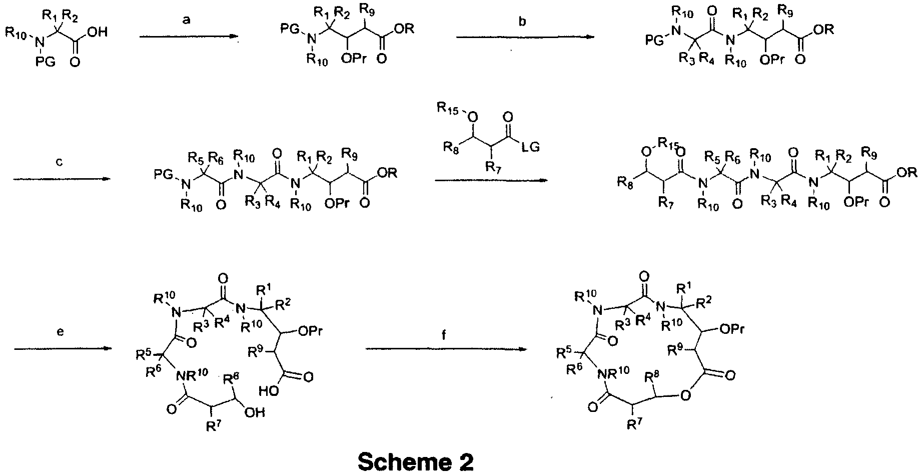

In Scheme 1 step (a), an *N*-protected amino acid bearing the side-chains R₁ and R₂ is condensed with an ester enolate bearing the side chain R₉ and the resulting intermediate 1,3-diketo-ester is then reduced to furnish a statine unit, wherein Pr is H or a removable alcohol-protecting group. In step (b), the N-protecting group is removed, and the statine is condensed with an *N*-protected amino acid bearing the side chains R₃ and R₄ to furnish a peptide isostere. In step (c), the *N*-protecting group is removed, and the peptide isostere is coupled with an *N*-protected amino acid bearing the side chains R₅ and R₆. In step (d), the *N*-protecting group is removed, and the resulting intermediate is coupled with a functionalised b-hydroxy acid derivative bearing the residues R₇ and R_{8,} the latter of which bears the Zinc-binding group in its terminus in protected or unprotected form; R₁₅ is a temporary blocking group which can be removed to produce a compound wherein R₁₅ is H; LG is a conventional leaving group, for example in the form of a chiral auxiliary as reported in Yurek-George, A. et al, *J. Am. Chem. Soc.* **2004,** *126,* 1030, or LG is an alcohol thus giving the free acid which allows for standard amide coupling. In step (e), the ester is hydrolysed, and cyclization is facilitated in step (f). Further chemical transformation of the terminal residue of R₈, for example ester hydrolysis to the corresponding carboxylic acid, a representative Zinc-binding group, may be conducted.

Compounds of the invention in which R₁₀ is other than hydrogen can be obtained either by alkylating a corresponding compound of the invention or intermediate in which R₁₀ is hydrogen or by using appropriately substituted starting materials.

As the skilled person will appreciate, when one of R₁, R₂, R₃, R₄, R₅, R₆, R₇, R₈, R₉, R₁₀ carries a functional group such as -OH, -SH, -NH₂ or -COOH, then it may be preferred for that group to be protected for one or more of the reaction steps following its introduction. In this case the group in question could be protected in a separate step after its introduction, or, it could be protected already at the time it is introduced. The skilled person will be aware of suitable protecting groups that can be used in this regard.

The AMD analogues thus may be salified by treatment with an appropriate acid or base. Racemic mixtures obtained by any of the above processes can be resolved by standard techniques, for example elution on a chiral chromatography column.

The skilled person will appreciate that various assays are suitable for testing for HDAC inhibition and may be used to measure the activity of a compound obtained from Scheme 1 or Scheme 2 compared to that of the known HDAC inhibitor SAHA. Thus, the IC₅₀ of a test compound against HDAC can, for example, be determined in an *in vitro* assay, and compared with the IC₅₀ of SAHA under the same assay conditions. If a test compound has an IC₅₀ value equal to or lower than that of SAHA it should be understood as having an HDAC inhibitory activity which is at least equal to that exhibited by SAHA.

Described herein is a process for selecting a compound which has an HDAC inhibitory activity which is at least equal to that exhibited by SAHA as defined above, wherein following completion of Scheme 1 or Scheme 2, the next step is a an *in vitro* HDAC assay. Typically, said assay comprises contacting a test compound and SAHA, at various concentrations, with diluted Hela Nuclear Extract to determine the IC₅₀ of the test compound and of SAHA against Hela Nuclear Extract. A test compound which has an IC₅₀ value measured against Hela Nuclear Extract which is equal to, or lower than, the IC₅₀ of SAHA under the same assay conditions should be understood as having an inhibitory activity which is at least equal to that exhibited by SAHA. Typically said assay is performed using a HDAC fluorescent activity assay kit (Biomol, UK) and the test compounds are reduced prior to analysis.

Also disclosed is a process for selecting a compound which has a human cancer cell growth inhibitory activity which is at least equal to that exhibited by SAHA, which process comprises preparing a compound of Structure III or IV via Scheme 1 or Scheme 2 as defined above followed by screening the thus obtained compound to measure its activity as a human cancer cell growth inhibitor.

The skilled person will appreciate that various assays are suitable for testing for human cancer cell growth inhibition and may be used to measure the activity of a compound obtained from step (viii) compared to that of SAHA. Thus, the IC₅₀ of a test compound against human cancer cell growth can, for example, be determined in an *in vitro* assay, and compared with the IC₅₀ of SAHA under the same assay conditions. If a test compound has an IC₅₀ value equal to or lower than that of SAHA it should be understood as having an inhibitory activity which is at least equal to that exhibited by SAHA. Typically in this embodiment this step comprises an *in vitro* assay which comprises contacting a test compound and SAHA, at various concentrations, with an MCF7 breast, HUT78 T-cell leukaemia, A2780 ovarian, PC3 or LNCAP prostate cancer cell line to determine the IC₅₀ of the test compound and of SAHA against the cell line. A test compound which has an IC₅₀ value measured against any of these cell lines which is equal to, or lower than, the IC₅₀ of SAHA under the same assay conditions should be understood as having an inhibitory activity at least equal to that of SAHA. Typically in this embodiment, said assay is performed using the CyQuant™ assay system (Molecular Probes, Inc. USA).

Also disclosed is a process for selecting a compound which has an anti-inflammatory activity which is at least equal to that exhibited by SAHA, which process comprises preparing a compound of Structure III or IV via Scheme 1 or Scheme 2 as defined above followed by screening the thus obtained compound to measure its anti-inflammatory activity.

The skilled person will appreciate that various assays are suitable for assessing the anti-inflammatory activity of a compound. The anti-inflammatory activity of a test compound relative to SAHA may, for example, be determined by measuring the activity of a compound in inhibiting the production of TNFα from peripheral blood mononuclear cells (PBMCs) relative to SAHA. Thus, the ability of a test compound to inhibit the production of TNFα from PBMCs can, for example, be determined in an assay, and compared with the activity of SAHA under the same assay conditions. If a test compound has an *in vitro* inhibitory activity of TNFα production which is equal to or higher than that of SAHA under the same assay conditions it should be understood as having an anti-inflammatory activity which is at least equal to that exhibited by SAHA. Typically this step is performed using the Quantikine^{®} Human-α assay kit (R&D systems, Abingdon UK).

The anti-inflammatory activity of a test compound relative to SAHA may be determined by assessing the activity of a compound in inhibiting inflammation in Balb/c mice relative to SAHA. If a test compound has an *in vivo* inhibitory activity which is equal to or higher than that of SAHA under the same test conditions it should be understood as having an anti-inflammatory activity which is at least equal to that exhibited by SAHA. Typically, in this embodiment this step is performed by assessing the *in vivo* activity of a test compound and of SAHA in inhibiting inflammation in Balb/c mice induced by a chemical challenge. Typically, said chemical challenge involves the topical administration to the mice of oxalazone or acetone. The compounds under investigation may be applied before or after the chemical challenge.

Also disclosed is a process for selecting a compound which has an activity in inducing a predominant G2/M phase arrest or cell death in MCF7 cells which is at least equal to that exhibited by SAHA, which process comprises preparing a compound of Structure III or IV via Scheme 1 or Scheme 2 as defined above followed by screening the thus obtained compound to measure activity in inducing a predominant G2/M phase arrest or cell death in MCF7 cells relative to SAHA.

The compounds of the present invention are found to be inhibitors of HDAC. The compounds of the present invention are therefore therapeutically useful.

The compounds of the invention and compositions comprising them may be administered in a variety of dosage forms. In one embodiment, a pharmaceutical composition comprising a compound of the invention may be formulated in a format suitable for oral, rectal, parenteral, intranasal or transdermal administration or administration by inhalation or by suppository. Typical routes of administration are parenteral, intranasal or transdermal administration or administration by inhalation.

The compounds of the invention can be administered orally, for example as tablets, troches, lozenges, aqueous or oily suspensions, dispersible powders or granules. Preferred pharmaceutical compositions of the invention are compositions suitable for oral administration, for example tablets and capsules.

The compounds of the invention may also be administered parenterally, whether subcutaneously, intravenously, intramuscularly, intrasternally, transdermally or by infusion techniques. The compounds may also be administered as suppositories.

The compounds of the invention may also be administered by inhalation. An advantage of inhaled medications are their direct delivery to the area of rich blood supply in comparison to many medications taken by oral route. Thus, the absorption is very rapid as the alveoli have an enormous surface area and rich blood supply and first pass metabolism is bypassed. A further advantage may be to treat diseases of the pulmonary system, such that delivering drugs by inhalation delivers them to the proximity of the cells which are required to be treated.

Also disclosed is an inhalation device containing such a pharmaceutical composition. Typically said device is a metered dose inhaler (MDI), which contains a pharmaceutically acceptable chemical propellant to push the medication out of the inhaler.

The compounds of the invention may also be administered by intranasal administration. The nasal cavity's highly permeable tissue is very receptive to medication and absorbs it quickly and efficiently, more so than drugs in tablet form. Nasal drug delivery is less painful and invasive than injections, generating less anxiety among patients. By this method absorption is very rapid and first pass metabolism is usually bypassed, thus reducing inter-patient variability. Also disclosed is an intranasal device containing such a pharmaceutical composition.

The compounds of the invention may also be administered by transdermal administration. The present invention therefore also provides a transdermal patch containing a compound of the invention, or a pharmaceutically acceptable salt thereof

The compounds of the invention may also be administered by sublingual administration. The present invention therefore also provides a sub-lingual tablet comprising a compound of the invention or a pharmaceutically acceptable salt thereof.

A compound of the invention is typically formulated for administration with a pharmaceutically acceptable carrier or diluent.

A compound of the invention may also be formulated with an agent which reduces degradation of the substance by processes other than the normal metabolism of the patient, such as anti-bacterial agents, or inhibitors of protease enzymes which might be the present in the patient or in commensural or parasite organisms living on or within the patient, and which are capable of degrading the compound.

Liquid dispersions for oral administration may be syrups, emulsions and suspensions.

Suspensions and emulsions may contain as carrier, for example a natural gum, agar, sodium alginate, pectin, methylcellulose, carboxymethylcellulose, or polyvinyl alcohol. The suspension or solutions for intramuscular injections may contain, together with the active compound, a pharmaceutically acceptable carrier, e.g. sterile water, olive oil, ethyl oleate, glycols, e.g. propylene glycol, and if desired, a suitable amount of lidocaine hydrochloride.

Solutions for injection or infusion may contain as carrier, for example, sterile water or preferably they may be in the form of sterile, aqueous, isotonic saline solutions.

The compounds of the present invention are therapeutically useful in the treatment or prevention of conditions mediated by HDAC. Accordingly, also disclosed is the use of a compound of the Structure III or IV, or a pharmaceutically acceptable salt thereof, in the manufacture of a medicament for use in the treatment or prevention of a condition materially affected by the activity of an HDAC. Also described is a method of treating a patient suffering from or susceptible to a condition mediated by HDAC, which method comprises administering to said patient an effective amount of a compound of Structure III or IV, or a pharmaceutically acceptable salt thereof.

In one embodiment the compounds of the present invention may be used in combination with another known inhibitor of HDAC, such as SAHA. In this embodiment, the combination product may be formulated such that it comprises each of the medicaments for simultaneous, separate or sequential use.

Therefore, also disclosed is the use of compounds according to Structure III or IV or a pharmaceutically acceptable salt thereof for use in the manufacture of a medicament for use in co-administration with another known inhibitor of HDAC, such as SAHA.

The compounds of the present invention can be used in both the treatment and prevention of cancer and can be used in a monotherapy or in a combination therapy. When used in a combination therapy, the compounds of the present invention are typically used together with small chemical compounds such as - cisplatins, anti-metabolites, DNA topoisomerase inhibitors, radiation, antibody-based therapies (for example Herceptin and Rituximab), anti-cancer vaccination, gene therapy, cellular therapies, hormone therapies or cytokine therapy.

In one embodiment of the invention a compound of the invention is used in combination with another chemotherapeutic or antineoplastic agent in the treatment of a cancer. Examples of such other chemotherapeutic or antineoplastic agents include mitoxantrone, vinca alkaloids for example vincristine and vinblastine, anthracycline antibiotics for example daunorubicin and doxorubicin, alkylating agents for example chlorambucil and melphalan, taxanes for example paclitaxel, antifolates for example methotrexate and tomudex, epipodophyllotoxins for example etoposide, camptothecins for example irinotecan and its active metabolite SN 38 and DNA methylation inhibitors for example the DNA methylation inhibitors disclosed in WO 02/085400.

According to the invention, therefore, products are provided which contain a compound of the invention and another chemotherapeutic or antineoplastic agent as a combined preparation for simultaneous, separate or sequential use in alleviating a cancer. Also disclosed is the use of an AMD as defined above or a pharmaceutically acceptable salt thereof in the manufacture of a medicament for use in the alleviation of cancer by co-administration with another chemotherapeutic or antineoplastic agent. The compound of the invention and the said other agent may be administrated in any order. In both these cases the compound of the invention and the other agent may be administered together or, if separately, in any order as determined by a physician.

HDAC is believed to contribute to the pathology and/or symptomology of several different diseases such that reduction of the activity of HDAC in a subject through inhibition of HDAC may be used to therapeutically address these disease states. Examples of various diseases that may be treated using the HDAC inhibitors of the present invention are described herein, and the use of compounds of the present invention described by Structure III or IV are included herein. It is noted that additional diseases beyond those disclosed herein may be later identified as applications of the compounds of the present invention, as the biological roles that HDAC play in various pathways becomes more fully understood.

One set of indications that HDAC inhibitors of the present invention may be used to treat are those involving undesirable or uncontrolled cell proliferation. Such indications include benign tumours, various types of cancers such as primary tumours and tumour metastasis, restenosis (e.g. coronary, carotid, and cerebral lesions), abnormal stimulation of endothelial cells (atherosclerosis), insults to body tissue due to surgery, abnormal wound healing, abnormal angiogenesis, diseases that produce fibrosis of tissue, repetitive motion disorders, disorders of tissues that are not highly vascularized, and proliferative responses associated with organ transplants. More specific indications for HDAC inhibitors include, but are not limited to prostate cancer, lung cancer, acute leukaemia, multiple myeloma, bladder carcinoma, renal carcinoma, breast carcinoma, colorectal carcinoma, neuroblastoma and melanoma.

In one embodiment, the compounds are provided for use in a method for treating diseases associated with undesired and uncontrolled cell proliferation. The method comprises administering to a subject suffering from uncontrolled cell proliferation a therapeutically effective amount of a HDAC inhibitor according to the present invention, such that said uncontrolled cell proliferation is reduced. The particular dosage of the inhibitor to be used will depend on the severity of the disease state, the route of administration, and related factors that can be determined by the attending physician. Generally, acceptable and effective daily doses are amounts sufficient to effectively slow or eliminate uncontrolled cell proliferation.

HDAC inhibitors according to the present invention may also be used in conjunction with other agents to inhibit undesirable and uncontrolled cell proliferation. Examples of other anti-cell proliferation agents that may be used in conjunction with the HDAC inhibitors of the present invention include, but are not limited to, retinoid acid and derivatives thereof, 2-methoxyestradiol, ANGIOSTATIN(TM) protein, ENDOSTATIN(TM) protein, suramin, squalamine, tissue inhibitor of metalloproteinase-I, tissue inhibitor of metalloproteinase-2, plasminogen activator inhibitor-1, plasminogen activator inhibitor-2, cartilage-derived inhibitor, paclitaxel, platelet factor 4, protamine sulfate (clupeine), sulfated chitin derivatives (prepared from queen crab shells), sulfated polysaccharide peptidoglycan complex (sp-pg), staurosporine, modulators of matrix metabolism, including for example, proline analogs ((1-azetidine-2-carboxylic acid (LACA), cishydroxyproline, d,1-3,4-dehydroproline, thiaproline), beta.-aminopropionitrile fumarate, 4-propyl-5-(4-pyridinyl)-2(3H)-oxazolone; methotrexate, mitoxantrone, heparin, interferons, 2 macroglobulin-serum, chimp-3, chymostatin, beta.-cyclodextrin tetradecasulfate, eponemycin; fumagillin, gold sodium thiomalate, d-penicillamine (CDPT), beta.-1-anticollagenase-serum, alpha.2-antiplasmin, bisantrene, lobenzarit disodium, n-(2-carboxyphenyl-4-chloroanthronilic acid disodium or "CCA", thalidomide; angostatic steroid, carboxyaminoimidazole; metalloproteinase inhibitors such as BB94. Other anti-angiogenesis agents that may be used include antibodies, preferably monoclonal antibodies against these angiogenic growth factors: bFGF, aFGF, FGF-5, VEGF isoforms, VEGF-C, HGF/SF and Ang-1/Ang-2. Ferrara N. and Alitalo, K. "Clinical application of angiogenic growth factors and their inhibitors" (1999) Nature Medicine 5:1359-1364.

Generally, cells in benign tumours retain their differentiated features and do not divide in a completely uncontrolled manner. A benign tumour is usually localized and nonmetastatic. Specific types of benign tumours that can be treated using HDAC inhibitors of the present invention include hemangiomas, hepatocellular adenoma, cavernous haemangioma, focal nodular hyperplasia, acoustic neuromas, neurofibroma, bile duct adenoma, bile duct cystanoma, fibroma, lipomas, leiomyomas, mesotheliomas, teratomas, myxomas, nodular regenerative hyperplasia, trachomas and pyogenic granulomas.

In the case of malignant tumors, cells become undifferentiated, do not respond to the body's growth control signals, and multiply in an uncontrolled manner. Malignant tumors are invasive and capable of spreading to distant sites (metastasizing). Malignant tumors are generally divided into two categories: primary and secondary. Primary tumors arise directly from the tissue in which they are found. Secondary tumors, or metastases, are tumors that originated elsewhere in the body but have now spread to distant organs. Common routes for metastasis are direct growth into adjacent structures, spread through the vascular or lymphatic systems, and tracking along tissue planes and body spaces (peritoneal fluid, cerebrospinal fluid, etc.).

Specific types of cancers or malignant tumors, either primary or secondary, that can be treated using the HDAC inhibitors of the present invention include, but are not limited to, leukaemia, breast cancer, skin cancer, bone cancer, prostate cancer, liver cancer, lung cancer, brain cancer, cancer of the larynx, gallbladder, pancreas, rectum, parathyroid, thyroid, adrenal, neural tissue, head and neck, colon, stomach, bronchi, kidneys, basal cell carcinoma, squamous cell carcinoma of both ulcerating and papillary type, metastatic skin carcinoma, osteo sarcoma, Ewing's sarcoma, veticulum cell sarcoma, myeloma, giant cell tumor, small-cell lung tumor, gallstones, islet cell tumor, primary brain tumor, acute and chronic lymphocytic and granulocytic tumors, hairy-cell tumor, adenoma, hyperplasia, medullary carcinoma, pheochromocytoma, mucosal neuronms, intestinal ganglloneuromas, hyperplastic corneal nerve tumor, marfanoid habitus tumor, Wilms' tumor, seminoma, ovarian tumor, leiomyomater tumor, cervical dysplasia and in situ carcinoma, neuroblastoma, retinoblastoma, soft tissue sarcoma, malignant carcinoid, topical skin lesion, mycosis fungoide, rhabdomyosarcoma, Kaposi's sarcoma, osteogenic and other sarcoma, malignant hypercalcemia, renal cell tumor, polycythermia vera, adenocarcinoma, glioblastoma multiforme, leukemias, lymphomas, malignant melanomas, epidermoid carcinomas, and other carcinomas and sarcomas.

The HDAC inhibitors of the present invention may also be used to treat abnormal cell proliferation due to insults to body tissue during surgery. These insults may arise as a result of a variety of surgical procedures such as joint surgery, bowel surgery, and cheloid scarring. Diseases that produce fibrotic tissue include emphysema. Repetitive motion disorders that may be treated using the present invention include carpal tunnel syndrome. An example of a cell proliferative disorder that may be treated using the invention is a bone tumor.

Proliferative responses associated with organ transplantation that may be treated using HDAC inhibitors of the invention include proliferative responses contributing to potential organ rejections or associated complications. Specifically, these proliferative responses may occur during transplantation of the heart, lung, liver, kidney, and other body organs or organ systems.

Abnormal angiogenesis that may be may be treated using this invention include those abnormal angiogenesis accompanying rheumatoid arthritis, ischemic-reperfusion related brain oedema and injury, cortical ischemia, ovarian hyperplasia and hypervascularity, (polycystic ovary syndrome), endometriosis, psoriasis, diabetic retinopathy, and other ocular angiogenic diseases such as retinopathy of prematurity (retrolental fibroplastic), macular degeneration, corneal graft rejection, neuroscular glaucoma and Oster Webber syndrome.

Examples of diseases associated with uncontrolled angiogenesis that may be treated according to the present invention include, but are not limited to retinal/choroidal neovascularization and corneal neovascularization. Examples of retinal/choroidal neovascularization include, but are not limited to, Bests diseases, myopia, optic pits, Stargarts diseases, Pagets disease, vein occlusion, artery occlusion, sickle cell anemia, sarcoid, syphilis, pseudoxanthoma elasticum carotid apo structive diseases, chronic uveitis/vitritis, mycobacterial infections, Lyme's disease, systemic lupus erythematosus, retinopathy of prematurity, Eales disease, diabetic retinopathy, macular degeneration, Bechets diseases, infections causing a retinitis or chroiditis, presumed ocular histoplasmosis, pars planitis, chronic retinal detachment, hyperviscosity syndromes, toxoplasmosis, trauma and post-laser complications, diseases associated with rubesis (neovascularization of the angle) and diseases caused by the abnormal proliferation of fibrovascular or fibrous tissue including all forms of proliferative vitreoretinopathy. Examples of corneal neovascularization include, but are not limited to, epidemic keratoconjunctivitis, Vitamin A deficiency, contact lens overwear, atopic keratitis, superior limbic keratitis, pterygium keratitis sicca, sjogrens, acne rosacea, phylectenulosis, diabetic retinopathy, retinopathy of prematurity, corneal graft rejection, Mooren ulcer, Terrien's marginal degeneration, marginal keratolysis, polyarteritis, Wegener sarcoidosis, Scleritis, periphigoid radial keratotomy, neovascular glaucoma and retrolental fibroplasia, syphilis, Mycobacteria infections, lipid degeneration, chemical burns, bacterial ulcers, fungal ulcers, Herpes simplex infections, Herpes zoster infections, protozoan infections and Kaposi sarcoma.

Chronic inflammatory diseases associated with uncontrolled angiogenesis may also be treated using HDAC inhibitors of the present invention. Chronic inflammation depends on continuous formation of capillary sprouts to maintain an influx of inflammatory cells. The influx and presence of the inflammatory cells produce granulomas and thus maintains the chronic inflammatory state. Inhibition of angiogenesis using a HDAC inhibitor alone or in conjunction with other anti-inflammatory agents may prevent the formation of the granulosmas and thus alleviate the disease. Examples of chronic inflammatory diseases include, but are not limited to, inflammatory bowel diseases such as Crohn's disease and ulcerative colitis, psoriasis, sarcoidosis, and rheumatoid arthritis.

Inflammatory bowel diseases such as Crohn's disease and ulcerative colitis are characterized by chronic inflammation and angiogenesis at various sites in the gastrointestinal tract. For example, Crohn's disease occurs as a chronic transmural inflammatory disease that most commonly affects the distal ileum and colon but may also occur in any part of the gastrointestinal tract from the mouth to the anus and perianal area. Patients with Crohn's disease generally have chronic diarrhoea associated with abdominal pain, fever, anorexia, weight loss and abdominal swelling. Ulcerative colitis is also a chronic, nonspecific, inflammatory and ulcerative disease arising in the colonic mucosa and is characterized by the presence of bloody diarrhoea. These inflammatory bowel diseases are generally caused by chronic granulomatous inflammation throughout the gastrointestinal tract, involving new capillary sprouts surrounded by a cylinder of inflammatory cells. Inhibition of angiogenesis by these inhibitors should inhibit the formation of the sprouts and prevent the formation of granulomas. Inflammatory bowel diseases also exhibit extra intestinal manifestations, such as skin lesions. Such lesions are characterized by inflammation and angiogenesis and can occur at many sites other the gastrointestinal tract. Inhibition of angiogenesis by HDAC inhibitors according to the present invention can reduce the influx of inflammatory cells and prevent lesion formation.

Sarcoidosis, another chronic inflammatory disease, is characterized as a multisystem granulomatous disorder. The granulomas of this disease can form anywhere in the body. Thus, the symptoms depend on the site of the granulomas and whether the disease is active. The granulomas are created by the angiogenic capillary sprouts providing a constant supply of inflammatory cells. By using HDAC inhibitors according to the present invention to inhibit angiogenesis, such granulomas formation can be inhibited. Psoriasis, also a chronic and recurrent inflammatory disease, is characterized by papules and plaques of various sizes. Treatment using these inhibitors alone or in conjunction with other anti-inflammatory agents should prevent the formation of new blood vessels necessary to maintain the characteristic lesions and provide the patient relief from the symptoms.

Rheumatoid arthritis (RA) is also a chronic inflammatory disease characterized by non-specific inflammation of the peripheral joints. It is believed that the blood vessels in the synovial lining of the joints undergo angiogenesis. In addition to forming new vascular networks, the endothelial cells release factors and reactive oxygen species that lead to pannus growth and cartilage destruction. The factors involved in angiogenesis may actively contribute to, and help maintain, the chronically inflamed state of rheumatoid arthritis. Treatment using HDAC inhibitors according to the present invention alone or in conjunction with other anti-RA agents may prevent the formation of new blood vessels necessary to maintain the chronic inflammation.

The compounds of the present invention can further be used in the treatment of cardiac/vasculature diseases such as hypertrophy, hypertension, myocardial infarction, reperfusion, ischaemic heart disease, angina, arrythmias, hypercholesterolemia, atherosclerosis and stroke. The compounds can further be used to treat neurodegenerative disorders/CNS disorders such as acute and chronic neurological diseases, including stroke, Huntington's disease, Amyotrophic Lateral Sclerosis and Alzheimer's disease.

The compounds of the present invention can also be used as antimicrobial agents, for example antibacterial agents. The invention therefore also provides a compound for use in the treatment of a bacterial infection. The compounds of the present invention can be used as anti-infectious compounds against viral, bacterial, fungal and parasitic infections. Examples of infections include protozoal parasitic infections (including plasmodium, cryptosporidium parvum, toxoplasma gondii, sarcocystis neurona and Eimeria sp.)

The compounds of the present invention are particularly suitable for the treatment of undesirable or uncontrolled cell proliferation, preferably for the treatment of benign tumours/hyperplasias and malignant tumors, more preferably for the treatment of malignant tumors and most preferably for the treatment of CCL, breast cancer and T-cell lymphoma.

In a preferred embodiment of the invention, the compounds of the invention are for use to alleviate cancer, cardiac hypertrophy, chronic heart failure, an inflammatory condition, a cardiovascular disease, a haemoglobinopathy, a thalassemia, a sickle cell disease, a CNS disorder, an autoimmune disease, diabetes, osteoporosis, MDS, benign prostatic hyperplasia, oral leukoplakia, a genentically related metabolic disorder, an infection, Rubens-Taybi, fragile X syndrome, or alpha-1 antitrypsin deficiency, or to accelerate wound healing, to protect hair follicles or as an immunosuppressant.

Typically, said inflammatory condition is a skin inflammatory condition (for example psoriasis, acne and eczema), asthma, chronic obstructive pulmonary disease (COPD), rheumatoid arthritis (RA), inflammatory bowel disease (IBD), Crohn's disease or colitis.

Typically, said cancer is chronic lymphocytic leukaemia, breast cancer, prostate cancer, ovarian cancer, mesothelioma or T-cell lymphoma.

Typically, said cardiovascular disease is hypertension, myocardial infarction (MI), ischemic heart disease (IHD) (reperfusion), angina pectoris, arrhythmia, hypercholesterolemia, hyperlipidaemia, atherosclerosis, stroke, myocarditis, congestive heart failure, primary and secondary i.e. dilated (congestive) cardiomyopathy, hypertrophic cardiomyopathy, restrictive cardiomyopathy, peripheral vascular disease, tachycardia, high blood pressure or thrombosis.

Typically, said genentically related metabolic disorder is cystic fibrosis (CF), peroxisome biogenesis disorder or adrenoleukodystrophy.

Typically, the compounds of the invention are used as an immunosuppressant following organ transplant.

Typically, said infection is a viral, bacterial, fungal or parasitic infection, in particular an infection by S aureus, P acne, candida or aspergillus.

Typically, said CNS disorder is Huntingdon's disease, Alzheimer's disease, multiple sclerosis or amyotrophic lateral sclerosis.

In this embodiment, the compounds of the invention may be for use to alleviate cancer, cardiac hypertrophy, chronic heart failure, an inflammatory condition, a cardiovascular disease, a haemoglobinopathy, a thalassemia, a sickle cell disease, a CNS disorder, an autoimmune disease, diabetes or osteoporosis, or are used as an immunosuppressant.

The compounds of the invention may also be used to alleviate chronic lymphocytic leukaemia, breast cancer, prostate cancer, ovarian cancer, mesothelioma, T-cell lymphoma, cardiac hypertrophy, chronic heart failure or a skin inflammatory condition, in particular psoriasis, acne or eczema.

The compounds of the present invention can be used in the treatment of animals, preferably in the treatment of mammals and more preferably in the treatment of humans.

The compounds of the invention may, where appropriate, be used prophylactically to reduce the incidence of such conditions.

A therapeutically effective amount of a compound of the invention is administered to a patient. A typical dose is from about 0.001 to 50 mg per kg of body weight, according to the activity of the specific compound, the age, weight and conditions of the subject to be treated, the type and severity of the disease and the frequency and route of administration.

### Examples

### Compound VII: tert-Butyl 5-((2R,6R,9R,12R,13S)-13-hydroxy-12-isopropyl-9-methyl-4,7,10,15-tetraoxo-6-(thiazol-4-ylmethyl)-1-oxa-5,8,11-triazacyclopentadecan-2-yl)pentanoate(reference compound) and Compound VIII: 5-((2R,6R,9R,12R,13S)-13-hydroxy-12-isopropyl-9-methyl-4,7,10,15-tetraoxo-6-(thiazol-4-ylmethyl)-1-oxa-5,8,11-triazacyclopentadecan-2-yl)pentanoic acid

### (9): (R)-8-tert-butoxy-3-hydroxy-8-oxooctanoic acid

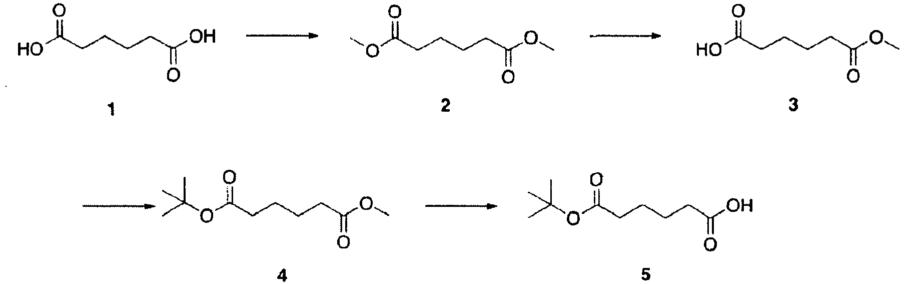

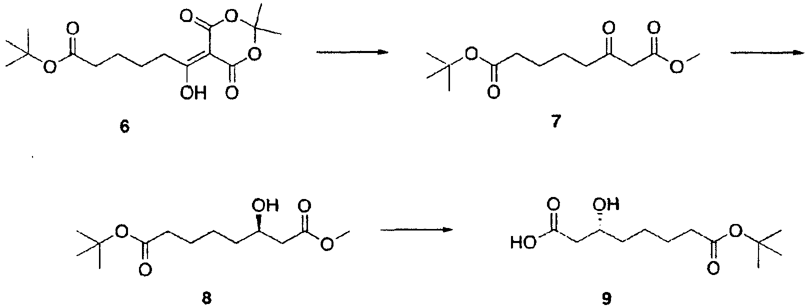

### (2): Dimethyl adipate

A mixture of adipic acid **1** (29.2g, 0.2mol) and SOCl₂ (119g) in MeOH (200mL) were stirred under reflux for 3h. The volatiles were removed in *vacuo.* The residue was dissolved in CH₂Cl₂ (200mL), washed with brine (100mL x 2), dried over MgSO₄ and concentrated in *vacuo* to obtain **2** (31.3g, 90%) as a colourless oil, which was used directly for next step.

### (3): 6-Methoxy-6-oxohexanoic Acid

A mixture of **2** (2.86 g, 1.64 mmol) and KOH (0.921 g, 1.64 mmol) in MeOH (50mL) were stirred at rt for 48 h, then the solvent was removed in *vacuo.* The residue was dissolved in water (50mL) and extracted with diethyl ether (50mL x 3). The aqueous layer was acidified with 1 N HCl to pH = 4-5 and extracted with EtOAc (50mL × 6). The combined EtOAc layers were dried over MgSO₄, concentrated in *vacuo* and dried on the high-vacuum pump to afford **3** (1.97g, 75%) as a colourless oil.
¹H NMR (500 MHz, CDCl₃) δ_{H}: 3.68 (s, 3H), 2.31 - 2.42 (m, 4H), 1.62 - 1.75 (m, 4H).

### (4): tert-Butyl methyl adipate

To a solution of **3** (8.0g, 49.4mmol) in CH₂Cl₂ (60mL) were added oxalyl chloride (13.1mL, 149.8mmol) and two drops of DMF. The reaction was stirred for 1 h and then concentrated in *vacuo.* The residue was dried under vacuum for 30 minutes.
At 0°C to a solution of the crude chloride (9.79 g) in CH₂Cl₂ (70mL) was added pyridine (6.0mL, 77.6mmol) dropwise and *t*-BuOH (5.0mL, 87mmol). The mixture was warmed to rt and stirred for 1 h. Then the mixture was washed with water, dried over MgSO₄, filtered and evaporated. The residue was purified by silica gel column chromatography eluting with petroleum ether/EtOAc (30/1) to obtain **4** (6.5g, 60%) as a colorless oil.
¹H NMR (500 MHz, CDCl₃) δ_{H}: 3.66 (s, 3H), 2.32 (t, *J*=7.0Hz, 2H), 2.22 (t, *J*=7.0Hz, 2H), 1.36 - 1.49 (m, 4H), 1.43 (s, 9H).

### (5): 6-tert-Butoxy-6-oxohexanoic acid

A mixture of **4** (4.325g, 20mmol) and KOH (1.120g, 20mmol) in THF/H₂O (50mL, 1:1) were stirred at rt overnight. The solvents were evaporated and the residue was dissolved in water (50mL). The aqueous layer was washed with diethyl ether (50mL × 3) and then acidified with 1 N HCl to pH = 4-5 and extracted with EtOAc (40mL × 5). The combined EtOAc layers were dried over MgSO₄ and concentrated in *vacuo* to obtain **5** (2.0g, 50%) as a colourless oil, which was used directly for next step.
¹H NMR (400 MHz, CDCl₃) δ_{H}: 2.38 (t, *J*=6.8Hz, 2H), 2.24 (t, *J*=6.8Hz, 2H), 1.58 - 1.73 (m, 4H), 1.44 (s, 9H).

### (6): tert-Butyl 6-(2,2-dimethyl-4,6-dioxo-1,3-dioxan-5-ylidene)-6-hydroxyhexanoate

A mixture of **5** (2.5g, 12.3mmol), DCC (3.3g, 16mmol), 2,2-dimethyl-1,3-dioxane-4,6-dione (2.67g, 18.5mmol) and DMAP (0.015g, 0.12mmol) were dissolved in CH₂Cl₂ (100mL) and stirred at rt overnight. After removal of the solvent the residue was dissolved in EtOAc (30mL) and filtered. The organic phase was washed with water (10mL) and brine (10mL), dried over MgSO₄, and concentrated in *vacuo* to afford **6** (3.23g, 80%) as a white solid, which was used directly for next step.

### (7): 8-tert-butyl 1-methyl 3-oxooctanedioate

A solution of **6** (3.12g, 9.5mmol) in MeOH (40mL) was stirred under reflux for 18h. The solvent was removed in *vacuo.* The residue was purified by silica gel column chromatography eluting with petroleum ether/EtOAc (30/1) to afford **7** (1.71g, 70%) as a colourless oil.
¹H NMR (500 MHz, CDCl₃) δ_{H}: 3.74 (s, 3H), 3.45 (s, 2H), 2.56 (t, *J*=7.0Hz, 2H), 2.22 (t, *J*=7.0Hz, 2H), 1.54 - 1.69 (m, 4H), 1.44 (s, 9H).

### (8): (R)-8-tert-butyl 1-methyl 3-hydroxyoctanedioate

A mixture of 7 (1.6g, 6.1mmol) and (*R*)-BINAP-Ru (50 mg) in MeOH (40mL) was stirred at 60°C under 100atm of H₂ for 3 days. The solution was evaporated and purified by silica gel column chromatography eluting with petroleum ether/EtOAc (30/1 to 5/1) to afford **8** (1.21 g, 75%) as a colourless oil.
¹H NMR (500 MHz, CDCl₃) δ_{H}: 3.96 - 4.05 (m, 1H), 3.72 (s, 3H), 2.51 (dd, *J*=16.8, 2.8Hz, 1H), 2.22 (dd, *J*=16.8, 9.0Hz, 1H), 2.23 (t, *J*=7.5Hz, 2H), 1.72-2.18 (m, 6H), 1.44 (s, 9H).

### (9): (R)-8-tert-butoxy-3-hydroxy-8-oxooctanoic acid

A mixture of 8 (0.51 g, 1.96mmol) and LiOH·H₂O (0.118g, 2.81 mmol) in a solution of THF (15mL) and water (5mL) was stirred at 0°C for 1 h. THF was evaporated and the aqueous layer was extracted with ether (10mL × 2), acidified with 1N HCl to pH = 4-5. The resulting solution was extracted with EtOAc (20mL × 4), dried over MgSO₄, concentrated and purified by silica gel column chromatography eluting with petroleum ether/EtOAc (8/1) to afford 9 (416 mg, yield: 90%) as a white solid.
¹H NMR (500 MHz, CDCl₃) δ_{H}: 4.01 - 4.09 (m, 1H), 2.57 (dd, *J*=17.0, 3.5Hz, 1H), 2.48 (dd, *J*=17.0, 8.5Hz, 1 H), 2.24 (t, *J*=7.0Hz, 2H), 1.33 - 1.69 (m, 6H), 1.44 (s, 9H). MS (ES⁺) 269 (100%, [M+Na]⁺). [a]²⁰_{D} -13 (CHCl₃, *c* 0.1).

### Compound VII: tert-Butyl 5-((2R,6R,9R,12R,13S)-13-hydroxy-12-isopropyl-9-methyl-4,7,10,15-tetraoxo-6-(thiazol-4-ylmethyl)-1-oxa-5,8,11-triazacyclopentadecan-2-yl)pentanoate (reference compound) and Compound VIII: 5-((2R,6R,9R,12R,13S)-13-hydroxy-12-isopropyl-9-methyl-4,7,10,15-tetraoxo-6-(thiazol-4-ylmethyl)-1-oxa-5,8,11-triazacyclopentadecan-2-yl)pentanoic acid

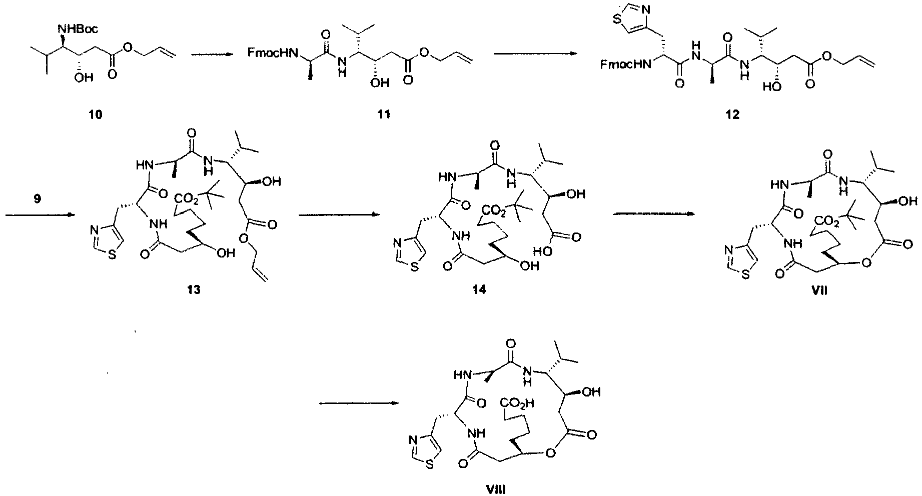

### (11): (3S,4R)-4-[(R)-2-(9H-Fluoren-9-ylmethoxycarbonylamino)-propionylamino]-3-hydroxy-5-methyl-hexanoic acid allyl ester

To a solution of compound 10 (2.88g, 9.57mmol, 1eq (prepared according to the procedure in Doi, T. et al, *Tet. Lett.* 2006, 47, 1177)) in MeCN (50mL) was added TFA (10%v/v, 5mL) dropwise at rt under Ar(g). The solution was stirred at rt for 2h, then the solvent was removed *in vacuo.* The reaction mixture was then co-evaporated with MeCN (3 x 15mL) before being dried under high vacuum for 2h.

To a solution of Fmoc-D-alanine (3.13g, 10.05mmol, 1.05 eq) in CH₂Cl₂ (100mL) at 0°C was added PyBOP (5.48g, 10.5mmol, 1.1eq) and DIPEA (5mL, 28.7mmol, 3 eq) under Ar(g). The crude amine, dissolved in MeCN (100mL), was added *via* cannula, and the reaction mixture was then left to warm to rt before being concentrated *in vacuo* after 2h. Purification by flash column chromatography (eluant 10-60% EtOAc/Hexane) furnished **11** (3.8g, 7.68mmol, 80%) as a white solid.
¹H NMR (400MHz, 9:1 CDCl₃/CD₃OD) δ_{H}: 7.75 (d, *J*=8.0Hz, 1H), 7.66 (d, *J*=7.5Hz, 2H), 7.63 (d, *J*=7.8Hz, 1H), 7.50 (d, *J*=7.5Hz, 2H), 7.30 (t, *J*=7.5Hz, 2H), 7.21 (t, *J*=7.5Hz, 2H), 5.72 - 5.86 (m, 1H), 5.20 (d, *J*=17.2Hz, 1H), 5.12 (d, J=10.5Hz, 1H), 4.49 (d, *J*=5.2Hz, 2H), 4.24 - 4.36 (m, 2H), 4.00 - 4.16 (m, 2H), 3.92 (td, *J*=8.5, 2.9Hz, 1H), 3.64 - 3.75 (m, 2H), 2.43 (d, *J*=15.3Hz, 1H), 2.32 (dd, *J*=15.6, 9.2Hz, 1H), 1.90 - 2.05 (m, 1H), 1.25 (d, *J*=7.1Hz, 3H), 0.79 (d, *J*=6.8Hz, 3H), 0.78 (d, *J*=6.8Hz, 3H). MS (ES⁺) 1012 (40%, [2M+Na]⁺), 517 (100%, [M+Na]⁺).

### (12): (3S,4R)-4-{(R)-2-[(R)-2-(9H-Fluoren-9-ylmethoxycarbonylamino)-3-thiazol-4-yl-propionylamino]-propionylamino}-3-hydroxy-5-methyl-hexanoic acid allyl ester

To a solution of **11** (1.46g, 2.95mmol, 1eq,) in MeCN (10mL) and THF (7mL) was added Et₂NH (10%v/v, 1.7mL) dropwise at rt under Ar(g). The solution was stirred at rt for 2h, and the solvent was removed *in vacuo.* The excess of amine was co-evaporated with MeCN (3 x 30mL) before the reaction mixture was dried under high vacuum for 2h.

To a solution of Fmoc-D-4-thiazoylalanine (1.22g, 3.10mmol, 1.05eq) in CH₂Cl₂ (25mL) at 0°C was added PyBOP (1.69g, 3.20mmol, 1.1eq) and DIPEA (1.3mL, 7.4mmol, 2.5eq) under Ar(g). The crude amine, dissolved in MeCN (25mL) was added *via* cannula, and the reaction mixture was then left to warm to rt overnight before being concentrated *in vacuo.* Purification by flash column chromatography (eluant 1-4% MeOH/CH₂Cl₂) furnished **12** (1.91g, 2.94mmol, 99%) as a white solid.
¹H NMR (400 MHz, 9:1 CDCl₃/CD₃OD) δ_{H}: 8.79 (s, 1H), 7.67 (d, *J*=7.6Hz, 2H), 7.47 (br. s., 2H), 7.30 (t, *J*=7.6Hz, 2H), 7.21 (t, *J*=7.6Hz, 2H), 7.07 (br. s., 1H), 5.73 - 5.87 (m, 1H), 5.21 (dq, *J*=17.3, 1.6Hz, 1 H), 5.12 (dd, *J*=10.4, 1.3Hz, 1H), 4.50 (d, *J*=5.5Hz, 2H), 4.28 - 4.41 (m, 2H), 4.15 - 4.28 (m, 2H), 4.09 (t, *J*=6.5Hz, 1H), 3.97 (t, *J*=8.7Hz, 1 H), 3.64 - 3.72 (m, 1 H), 3.16 - 3.25 (m, 1H), 2.98 - 3.16 (m, 1H), 2.46 (d, *J*=15.2Hz, 1H), 2.32 (ddd, *J*=15.7, 9.7, 1.4Hz, 1H), 1.90 - 2.03 (m, 1H), 1.23 (d, *J*=6.7Hz, 3H), 0.78 (t, *J*=6.8Hz, 6H). MS (ES⁺) 672 (60%, [M+Na]⁺), 650 (100%, [M+H]⁺).

### (13): (R)-7-{(R)-1-[(R)-1-((1R,2S)-3-Allyloxycarbonyl-2-hydroxy-1-isopropyl-propylcarbamoyl)-ethylcarbamoyl]-2-thiazol-4-yl-ethylcarbamoyl}-6-hydroxy-heptanoic acid tert-butyl ester

To a solution of **12** (1.91g, 2.94mmol, 1eq,) in MeCN (10mL) and THF (7mL) was added Et₂NH (10% v/v, 1.7mL) dropwise at rt under Ar(g). The solution was stirred at rt for 3h, then the solvent was removed *in vacuo.* The excess of amine was co-evaporated with MeCN (3 x 50mL) before the reaction mixture was dried under high vacuum for 2 h.

To a solution of (R)-3-hydroxy-octanedioic acid 8-tert-butyl ester **9** (0.763g, 3.10mmol, 1.05eq, prepared by Chempartner Inc., Shanghai, China) in MeCN (25mL) at 0°C was added PyBOP (1.69g, 3.25mmol, 1.1eq) and N-ethyldiisopropylamine (1.3mL, 7.4mmol, 2.5 eq) under Ar(g). The crude amine, dissolved in THF (25mL) was added *via* cannula, and the reaction mixture was then left to warm to rt overnight before being filtered and then concentrated *in vacuo.* Purification by flash column chromatography (eluant 4-12% IPA/CH₂Cl₂) gave the **13** (1.92g, 2.93mmol, 99%) as a white solid.
¹H NMR (400 MHz, 9:1 CDCl₃/CD₃OD) δ_{H}: 8.81 (d, *J*=2.0Hz, 1H), 7.18 (d, *J*=1.8Hz, 1H), 6.96 (d, *J*=9.8Hz, 1H), 5.75 - 5.87 (m, *J*=17.3, 10.5, 5.5, 5.5Hz, 1H), 5.22 (dd, J=17.1, 1.3Hz, 4H), 5.13 (dd, *J*=10.3, 1.0Hz, 1H), 4.57 (dd, *J*=6.3, 5.5Hz, 1 H), 4.51 (d, *J*=5.5Hz, 3H), 4.17 (d, *J*=7.3Hz, 1 H), 4.00 (ddd, *J*=9.8, 7.3, 2.8Hz, 1 H), 3.80 - 3.88 (m, 1 H), 3.64 - 3.73 (m, 1 H), 3.19 - 3.24 (m, 2H), 2.46 (dd, *J*=15.8, 2.8Hz, 1H), 2.33 (dd, *J*=15.8, 9.5Hz, 1 H), 2.25 (dd, *J*=13.6, 3.3Hz, 1 H), 2.10 - 2.21 (m, 3H), 1.90 - 2.02 (m, 1 H), 1.34 (s, 9H), 1.24 - 1.58 (m, 4H), 1.14 (d, *J*=7.0Hz, 3H), 0.80 (d, *J*=6.8Hz, 6H). MS (ES⁺) 678 (100%, [M+Na]⁺), 656 (20%, [M+H]⁺).

### (14): (R)-7-{(R)-1-[(R)-1-((1R,2S)-3-Carboxy-2-hydroxy-1-isopropyl-propylcarbamoyl)-ethylcarbamoyl]-2-thiazol-4-yl-ethylcarbamoyl}-6-hydroxy-heptanoic acid tert-butyl ester

To a solution of **13** (1.92 mg, 2.94mmol, 1eq) in anhydrous MeOH (25mL) was added morpholine (297µL, 3.4mmol, 1.15eq) and Pd(PPh₃)₄ (68 mg, 0.06mmol, 0.02eq) at rt under Ar(g). The reaction mixture was stirred for 2h, then concentrated in *vacuo.* Purification by flash column chromatography (eluant 3-15% MeOH/CH₂Cl₂ + 0.05% formic acid) gave **14** (1.36g, 2.21mmol, 75%) as a white solid: ¹H NMR (400 MHz, 9:1 CDCl₃/CD₃OD) δ_{H}: 8.78 (d, *J*=1.8Hz, 1H), 7.95 (d, *J*=3.5Hz, 1H), 7.16 (d, *J*=1.7Hz, 1H), 4.51 - 4.58 (m, *J*=7.3, 4.6Hz, 1H), 4.13 (q, *J*=7.1Hz, 1H), 3.90 - 3.98 (m, 1 H), 3.60 - 3.70 (m, 2H), 3.18 - 3.28 (m, 1 H), 3.15 (dd, *J*=14.7, 7.6Hz, 1H), 2.40 (dd, *J*=16.0, 3.1Hz, 1H), 2.06 - 2.33 (m, 5H), 1.90 - 2.01 (m, 1 H), 1.32 (s, 9H), 1.20 - 1.54 (m, 6H), 1.14 (d, *J*=7.2Hz, 3H), 0.77 (d, *J*=6.8Hz, 6H). MS (ES⁺) 638 (100%, [M+Na]⁺).

### Compound VII: 5-((2R,6R,9R,12R,13S)-13-Hydroxy-12-isopropyl-9-methyl-4,7,10,15-tetraoxo-6-thiazol-4-ylmethyl-1-oxa-5,8,11-triaza-cyclopentadec-2-yl)-pentanoic acid tert-butyl ester

To a solution of MNBA (914 mg, 2.65mmol) and DMAP (649mg, 5.31 mmol) in CH₂Cl₂ (2L) was added dropwise a solution of **14** (1.36g, 2.21mmol) in CH₂Cl₂/THF (60:1, 320mL) over 6h under Ar(g). After a further 14h the reaction mixture was concentrated *in vacuo* to give a yellow solid. Purification by column chromatography on silica gel, using 1-6% MeOH/CH₂Cl₂ as eluant, gave a 3:1 mixture of inseparable diastereomers of **VII** (265 mg, 0.44mmol, 20%) as a white solid.
¹H NMR (400 MHz, 9:1 CDCl₃/CD₃OD, major isomer only) δ_{H}: 8.74 (d, *J*=1.9Hz, 1 H), 7.09 (d, *J*=1.8Hz, 1 H), 5.03 - 5.14 (m, 1 H), 4.37 (dd, *J*=8.0, 6.3Hz, 1 H), 3.91 - 4.05 (m, 2H), 3.72 (q, *J*=7.4Hz, 1 H), 3.33 (dd, *J*=9.4, 4.4Hz, 1H), 3.13 - 3.22 (m, 1H), 3.09 (dd, *J*=14.7, 8.3Hz, 1H), 2.17 - 2.39 (m, 4H), 1.96 - 2.15 (m, 3H), 1.36 - 1.58 (m, 4H), 1.32 (d, *J*=7.3Hz, 3H), 1.28 (s, 9H), 1.12 - 1.23 (m, 2H), 0.79 (d, *J*=6.8Hz, 6H). MS (ES⁺) 620 (100%, [M+Na]⁺).

### Compound VIII: 5-((2R,6R,9R,12R,13S)-13-Hydroxy-12-isopropyl-9-methyl-4,7,10,15-tetraoxo-6-thiazol-4-ylmethyl-1-oxa-5,8,11-triaza-cyclopentadec-2-yl)-pentanoic acid

At rt Et₃SiH (348mL, 2.18mmol) followed by TFA (3.5mL) were added to a flask containing **compound VII** (260mg, 0.436mmol). After 40 min, the reaction mixture was concentrated *in vacuo.* Purification by column chromatography on silica gel (2-10% MeOH/CH₂Cl₂ then + 0.05% formic acid)) gave both the minor diastereoisomer (52mg, 0.096mmol) and the desired product **compound VIII** (157mg, 0.29mmol, 89%) as a white solid.
¹H NMR (400 MHz, 9:1 CDCl₃/CD₃OD) δ_{H}: 8.68 (d, *J*=2.0Hz, 1H), 7.08 (d, *J*=1.9Hz, 1H), 5.08 - 5.18 (m, 1H), 4.44 (dd, *J*=8.2, 6.3Hz, 1 H), 3.85 - 4.01 (m, 2H), 3.81 (q, *J*=7.3Hz, 1 H), 3.40 (dd, *J*=9.4, 4.4Hz, 1 H), 3.17 (dd, *J*=14.6, 6.4Hz, 1H), 3.10 (dd, *J*=14.6, 8.3Hz, 1H), 2.22 - 2.41 (m, 3H), 2.16 (t, *J*=7.3Hz, 3H), 1.40 - 1.62 (m, 4H), 1.33 (d, *J*=7.3Hz, 3H), 1.16 - 1.28 (m, 2H), 0.81 (d, *J*=6.8Hz, 6H). MS (ES⁺) 563 (100%, [M+Na]⁺).

### Compound IX: Methyl 5-((2R,6R,9R,12R,13S)-13-hydroxy-12-isopropyl-9-methyl-4,7,10,15-tetraoxo-6-(thiazol-4-yl-methyl)-1-oxa-5,8,11-triazacyclopentadecan-2-yl)pentanoate (reference compound)

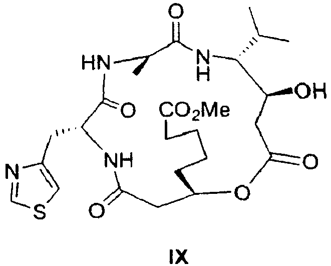

At 0°C to a solution **compound VIII** (10 mg, 0.018mmol) in CH₂Cl₂ (0.2mL) and THF (2 drops) was added a mixture containing cyanuric chloride (1.1mg, 0.006mmol), *N*-methylmorpholine (2.2mL, 0.02mmol), DMAP (0.1 mg, 0.0002mmol) and hydroxylamine hydrochloride (1.4mg, 0.02mmol) in CH₂Cl₂ (0.2mL). The reaction was allowed to warm to rt and stirred over night before being concentrated *in vacuo.* Initial purification by ion exchange column (SCX-3, eluant CH₂Cl₂/MeOH then 0.1-1 M NH₃ in MeOH) followed by purification by flash column chromatography (eluant 3-6% MeOH/CH₂Cl₂) gave **compound IX** (2.5 mg, 0.0045mmol, 25%) as a white solid.
¹H NMR (400MHz, 9:1 CDCl₃/CD₃OD) δ_{H}: 8.81 (s, 1H), 7.42 (d, *J*=9.5Hz, 1H), 7.18 (s, 1 H), 5.08 - 5.19 (m, 1 H), 4.41 - 4.49 (m, 1 H), 3.97 - 4.06 (m, 1 H), 3.82 (q, *J*=7.4Hz, 1 H), 3.60 (br. s., 1 H), 3.56 (s, 3H), 3.31 - 3.41 (m, 1 H), 3.09 - 3.23 (m, 2H), 2.08 - 2.43 (m, 6H), 1.42 - 1.65 (m, 4H), 1.35 (d, *J*=7.3Hz, 3H), 1.18 - 1.29 (m, 2H), 0.84 (d, *J*=6.7Hz, 6H); MS (ES⁺) 578 (60%, [M+Na]⁺), 650 (100%, [M+H]⁺).

### Compound X: (2S,6R,9R,12R,13S)-13-hydroxy-12-isopropyl-2-((E)-4-mercaptobut-1-enyl)-9-methyl-6-(thiazol-4-ylmethyl)-1-oxa-5,8,11-triazacyclopentadecane-4,710,15-tetraone (reference compound)

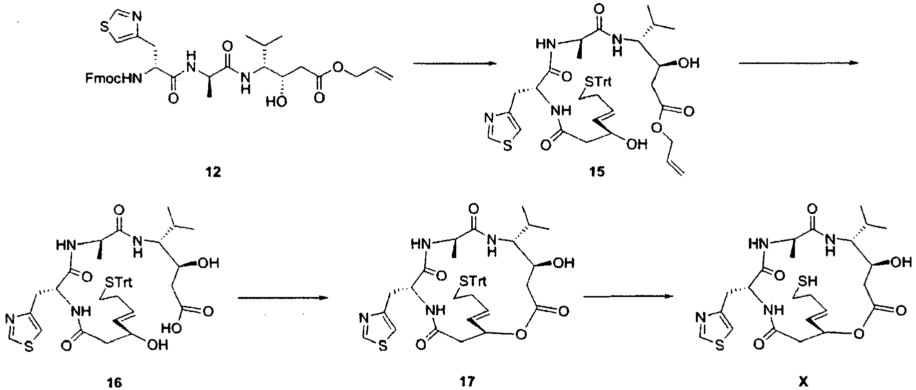

### (15): (3S,4R)-3-Hydroxy-4-{(R)-2-[(R)-2-((E)-(S)-3-hydroxy-7-tritylsulfanyl-hept-4-enoylamino)-3-thiazol-4-yl-propionylamino]-propionylamino}-5-methyl-hexanoic acid allyl ester

To a solution of **12** (761mg, 1.17mmol, 1eq,) in MeCN (9mL) and THF (1mL) was added Et₂NH (10% v/v, 1 mL) dropwise at rt under Ar(g). The solution was stirred at rt for 2h, then the solvent was removed *in vacuo.* The excess of amine was co-evaporated with MeCN (3 x 30mL) before the reaction mixture was dried under high vacuum for 2h.

To a solution of (E)-(S)-3-Hydroxy-7-tritylsulfanyl-hept-4-enoic acid (514mg, 1.23mmol, 1.05eq, prepared according to patent WO 2006/129105) in CH₂Cl₂ (20mL) at 0 °C was added PyBOP (670mg, 1.29mmol, 1.1eq) and N-ethyldiisopropylamine (0.51mL, 2.93mmol, 2.5eq) under Ar(g). The crude amine, dissolved in MeCN (20mL) was added *via* cannula, and the reaction mixture was then left to warm to rt overnight before being filtered and then concentrated *in vacuo.* Purification by flash column chromatography (eluant 2-10% IPA/CH₂Cl₂) gave **15** (860mg, 1.02mmol, 87%) as a white solid.
¹H NMR (400 MHz, 9:1 CDCl₃/CD₃OD) δ_{H}: 8.84 (s, 1H), 7.11 - 7.40 (m, 16H), 5.78 - 5.91 (m, 1 H), 5.48 (dd, *J*=15.2, 6.3Hz, 1 H), 5.36 (dd, *J*=15.4, 6.3Hz, 1 H), 5.26 (dd, *J*=17.2, 1.5Hz, 1H), 5.17 (dd, *J*=10.4, 1.3Hz, 1H), 4.60 (t, *J*=6.0Hz, 1H), 4.55 (d, *J*=5.6Hz, 2H), 4.30 (d, *J*=6.5Hz, 1H), 4.15 - 4.23 (m, 1H), 4.00 - 4.07 (m, 1H), 3.56 - 3.65 (m, 1H), 3.25 (d, *J*=5.9Hz, 2H), 2.46 - 2.54 (m, 1H), 2.37 (dd, *J*=15.8, 9.7Hz, 1H), 2.26 (d, *J*=7.5Hz, 2H), 2.11 - 2.19 (m, 2H), 1.94 - 2.07 (m, 3H), 1.15 (d, *J*=7.2Hz, 3H), 0.84 (d, *J*=6.8Hz, 6H). MS (ES⁺) 850 (100%, [M+Na]⁺).

### (16) (3S,4R)-3-Hydroxy-4-{(R)-2-[(R)-2-((E)-(S)-3-hydroxy-7-tritylsulfanyl-hept-4-enoylamino)-3-thiazol-4-yl-propionylamino]-propionylamino}-5-methyl-hexanoic acid

To a solution of **15** (790mg, 0.936mmol, 1eq) in anhydrous MeOH (10mL) was added morpholine (96µL, 1.08mmol, 1.15eq) and Pd(PPh₃)₄ (22mg, 0.02mmol, 0.02eq) at rt under Ar(g). The reaction mixture was stirred for 1.5h, then concentrated *in vacuo.* Purification by flash column chromatography (eluant 3-20% MeOH/CH₂Cl₂) gave **16** (620mg, 0.788mmol, 84%) as a white solid.
¹H NMR (400 MHz, 9:1 CDCl₃/CD₃OD) δ_{H}: 8.69 (d, *J*=2.0Hz, 1H), 7.03 - 7.33 (m, 16H), 5.43 (dd, *J*=15.4, 6.5Hz, 1H), 5.31 (dd, *J*=15.4, 6.3Hz, 1H), 4.53 (dd, *J*=7.0, 5.0H, 1 H), 4.25 (d, *J*=6.5Hz, 1 H), 4.11 (d, *J*=7.2Hz, 1 H), 3.90 (td, *J*=8.3, 3.3Hz, 1H), 3.72 - 3.81 (m, 1H), 3.18 (d, *J*=3.8Hz, 2H), 2.36 (dd, *J*=15.8, 3.2Hz, 1H), 2.16 - 2.26 (m, 3H), 2.06 - 2.14 (m, 2H), 1.90 - 2.02 (m, 3H), 1.11 (d, *J*=7.2Hz, 3H), 0.78 (d, *J*=6.7Hz, 6H). MS (ES⁺) 809 (100%, [M+Na]⁺), (ES⁻) 785 (100%, [M-H]⁻).

### (17): (2S,6R,9R,12R,13S)-13-Hydroxy-12-isopropyl-9-methyl-6-thiazol-4-ylmethyl-2-((E)-4-tritylsulfanyl-but-1-enyl)-1-oxa-5,8,11-triaza-cyclopentadecane-4,7,10,15-tetraone

To a solution of MNBA (320mg, 0.93mmol) and DMAP (227mg, 1.86mmol) in CH₂Cl₂ (450mL) was added dropwise a solution of **16** (610mg, 0.775mmol) in CH₂Cl₂/THF (45:1, 460mL) over 5h under Ar(g). After a further 6 days, the reaction mixture was concentrated *in vacuo* to give a yellow solid. Purification by column chromatography on silica gel (eluant 2-12% IPA/CH₂Cl₂) gave **17** (77mg, 0.010mmol, 13%) as a white solid.
¹H NMR (400 MHz, 9:1 CDCl₃CD₃OD) δ_{H}: 8.84 (s, 1 H), 7.07 - 7.41 (m, 16H), 5.44 - 5.62 (m, 2H), 5.29 (dd, *J*=15.5, 6.7Hz, 1H), 4.49 (t, *J*=7.0Hz, 1H), 4.07 - 4.15 (m, 1H), 3.94 (q, *J*=6.5Hz, 1H), 3.14 - 3.29 (m, 3H), 2.48 (dd, *J*=14.6, 11.6Hz, 1H), 2.27 - 2.42 (m, 3H), 2.11 (d, *J*=7.0Hz, 3H), 1.90 - 2.05 (m, 2H), 1.30 (d, *J*=7.3Hz, 3H), 0.88 (d, *J*=6.8Hz, 3H), 0.85 (d, *J*=6.8Hz, 3H). MS (ES⁺) 791 (100%, [M+Na]⁺).

### Compound X: (2S,6R,9R,12R,13S)-13-Hydroxy-12-isopropyl-2-((E)-4-mercapto-but-1-enyl)-9-methyl-6-thiazol-4-ylmethyl-1-oxa-5,8,11-triazacyclopentadecane-4,7,10,15-tetraone

To a solution of **17** (80mg, 0.10) in CH₂Cl₂ (1mL) was added Et₃SiH followed by formic acid (1 mL). After stirring at rt for 20h the reaction was concentrated *in vacuo.* Purification by column chromatography on silica gel (eluant 3-8% MeOH/CH₂Cl₂) gave the **compound X** (29mg, 0.055mmol, 53%) as a white solid.
¹H NMR (400 MHz, 9:1 CDCl₃/CD₃OD) δ_{H}: 8.71 (d, *J*=1.9Hz, 1H), 7.45 (d, *J*=9.1Hz, 1H), 7.10 (d, *J*=1.8Hz, 1H), 5.62 (dt, *J*=15.2, 6.8Hz, 1H), 5.35 - 5.53 (m, 2H), 4.49 (t, *J*=7.5Hz, 1 H), 4.02 (ddd, *J*=9.6, 5.8, 5.3Hz, 1 H), 3.76 - 3.86 (m, 1 H), 3.05 - 3.23 (m, 3H), 2.40 - 2.54 (m, 3H), 2.28 - 2.39 (m, 3H), 2.07 - 2.27 (m, 3H), 1.28 (d, *J*=7.3Hz, 3H), 0.82 (d, *J*=6.7Hz, 3H), 0.81 (d, *J*=6.6Hz, 3H); ¹³C NMR (100 MHz, 9:1 CDCl₃/CD₃OD) δ_{C}: 173.4, 172.0, 171.4, 170.5, 153.6, 152.0, 131.9, 128.9, 116.3, 71.7, 68.6, 59.2, 53.8, 52.6, 41.0, 40.0, 36.1, 32.2, 27.8, 23.4, 20.2, 16.8, 15.8. MS (ES⁺) 549 (100%, [M+Na]⁺), 527 (10%, [M+H]⁺).

### Compound XI: 5-((2R,6R,9R,12R,13S)-13-Hydroxy-12-isopropyl-9-methyl-4,7,10,15-tetraoxo-6-thiazol-4-ylmethyl-1-oxa-5.8.11-triaza-cyclopentadec-2-yl)-pentanoic acid hydroxyamide

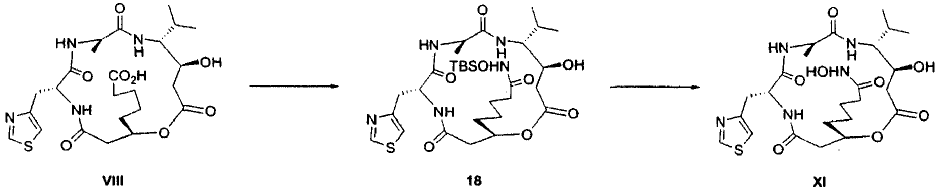

### (18): 5-((2R,6R,9R, 12R, 13S)-13-Hydroxy-12-isopropyl-9-methyl-4,7,10,15-tetraoxo-6-thiazol-4-ylmethyl-1-oxa-5,8,11-triaza-cyclopentadec-2-yl)-pentanoic acid tert-butyldimethylsilyloxy-amide

At rt to a solution of **compound VIII** (41 mg, 0.076mmol) in THF (2mL) was added a solution of EDAC.HCl (17.5mg, 0.091 mmol) in CH₂Cl₂ (1 mL) followed by a solution of H₂NO(TBS) (17mg, 0.114mmol) in CH₂Cl₂ (1mL). The reaction went cloudy, a further 6mL of CH₂Cl₂ was added. After 3 h the reaction mixture was concentrated *in vacuo.* Purification by column chromatography on silica gel (2-10% MeOH/CH₂Cl₂) gave the desired product **18** (23mg, 0.034mmol, 45%) as a white solid: ¹H NMR (400 MHz, 9:1 CDCl₃/CD₃OD) δ_{H}: 8.75 (s, 1H), 7.50 (d, *J*=9.7Hz, 1 H), 7.13 (s, 1 H), 5.06 - 5.19 (m, 1 H), 4.43 - 4.55 (m, 1H), 3.91 - 4.02 (m, 1H), 3.74 - 3.83 (m, 1H), 3.36 - 3.49 (m, 1H), 3.20 (dd, *J*=14.9, 9.0Hz, 1H), 3.13 (dd, *J*=13.4, 8.0Hz, 1 H), 2.20 - 2.44 (m, 4H), 2.07 - 2.20 (m, 1H), 1.92 - 2.05 (m, 2H), 1.43 - 1.60 (m, 4H), 1.36 (d, *J*=7.0Hz, 3H), 1.12 - 1.27 (m, 3H), 0.84 (d, *J*=6.2Hz, 3H), 0.83 (d, *J*=5.5Hz, 3H), 0.80 (s, 9H), -0.03 (s, 3H), -0.03 (s, 3H). MS (ES⁺) 692 (100%, [M+Na]⁺).

### Compound XI: 5-((2R,6R,9R,12R,13S)-13-Hydroxy-12-isopropyl-9-methyl-4,7,10,15-tetraoxo-6-thiazol-4-ylmethyl-1-oxa-5,8,11-triaza-cyclopentadec-2-yl)-pentanoic acid hydroxyamide

To **18** (23mg, 0.034mmol) was added formic acid (0.8mL) under Ar(g). After 50 min the solution was concentrated *in vacuo.* Purification by ion exchange cartridge (PE-AX, 2g, 0-80% MeOH/CH₂Cl₂) gave the hydroxamic acid **compound XI** (17.9mg, 0.032mmol, 95%) as a white solid: 1H NMR (400 MHz, 9:1 CDCl₃/CD₃OD) δ_{H}: 8.65 (d, *J*=1.9Hz, 1H), 7.08 (d, *J*=1.9Hz, 1H), 5.03 - 5.15 (m, 1H), 4.50 (dd, *J*=8.3, 6.7Hz, 1H), 3.91 (ddd, *J*=10.3, 7.2, 3.6Hz, 1H), 3.82 (q, *J*=7.3Hz, 1 H), 3.45 (dd, *J*=9.9, 3.6Hz, 1 H), 3.18 (dd, *J*=14.6, 6.5Hz, 1H), 3.09 (dd, *J*=14.6, 8.5Hz, 1H), 2.37 (dd, *J*=14.1, 11.5Hz, 1H), 2.28 (dd, *J*=15.9, 3.6Hz, 1H), 2.16 - 2.24 (m, 2H), 2.04 - 2.14 (m, 1H), 1.94 (t, *J*=7.0Hz, 2H), 1.38 - 1.59 (m, 4H), 1.32 (d, *J*=7.4Hz, 3H), 1.08 - 1.26 (m, 2H), 0.80 (d, *J*=7.0Hz, 3H), 0.78 (d, *J*=7.0Hz, 3H). MS (ES⁺) 578 (100%, [M+Na]⁺), 566 (30%, [M+H]⁺); (ES⁻) 554 (100%, [M-H]⁻).

### Compound XII:_ 5-((6R,9R,13R)-6-Methyl-5,8,11,15,18-pentaoxo-9-thiazol-4-ylmethyl-14-oxa-4,7,10,17-tetraaza-spiro[2.15]octadec-13-yl)-pentanoic acid tert-butyl ester(reference compound) and Compound XIII: 5-((6R,9R,13R)-6-Methyl-5,8,11,15,18-pentaoxo-9-thiazol-4-ylmethyl-14-oxa-4,7,10,17-tetraaza-spiro[2,15]octadec-13-yl)-pentanoic acid

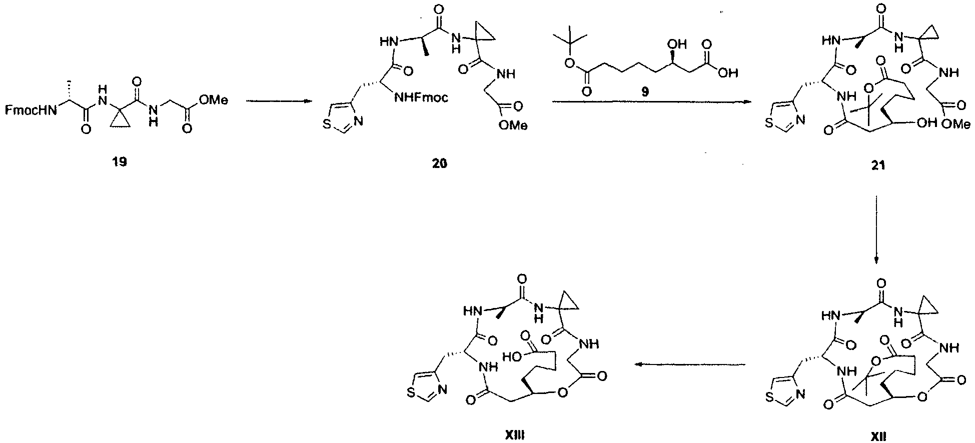

**(19)** was prepared using a well-known, conventional synthetic route starting from Fmoc 1-amino-1-cyclopropanecarboxylic acid, which was condensed with glycine methyl ester hydrochloride in the presence of PyBOP/*N,N-*diisopropylamine in a mixture of MeCN and CH₂Cl₂. The obtained methyl N-(Fmoc-1-amino-1-cyclopropylcarbonyl)glycine in acetonitrile was treated at rt with diethylamine for 1h to give methyl N-(1-amino-1-cyclopropylcarbonyl)glycine. This intermediate was finally reacted in a mixture of MeCN and CH₂Cl₂ with Fmoc-D-Ala-OH in the presence of PyBOP/DIEA to furnish **19.**

### (20): [(1-{(R)-2-[(R)-2-(9H-Fluoren-9-ylmethoxycarbonylamino)-3-thiazol-4-yl-propionylamino]-propionylamino}-cyclopropanecarbonyl)-amino]-acetic acid methyl ester

To a solution of the ({1-[(*R*)-2-(9H-Fluoren-9-ylmethoxycarbonylamino)-propionylamino]-cyclopropanecarbonyl}-amino)-acetic acid methyl ester **19** (500mg, 1.11mmol, 1eq (prepared by Chempartner, Shanghai, China)) in MeCN (22mL) was added Et₂NH (2.2mL, 10%v/v) dropwise at rt under Ar(g). The solution was stirred at rt for 2h, then the solvent was removed in *vacuo.* The excess of amine was co-evaporated with MeCN (3 x 10mL), then with a 1:5 mixture of CH₂Cl₂/hexane (10mL). A white solid was obtained and the flask was dried under high vacuum for 2h. To a solution of Fmoc-D-ThiazoylAla (480mg, 1.22mmol, 1.1eq) in MeCN (20mL) at 0°C was added PyBOP (634mg, 1.22mmol, 1.1eq) and N-ethyldiisopropylamine (482µL, 2.77mmol, 2.5eq) under Ar(g). The crude amine, dissolved in CH₂Cl₂ (20mL) was added to the mixture dropwise. The reaction mixture was then left to warm to rt overnight. The mixture was then concentrated in *vacuo* and the residue was further purified by silica gel column chromatography with EtOAc/MeOH (1:0 -> 49:1) to yield **20** as a white solid (650mg, 97%).
¹H NMR (400 MHz, CDCl₃ + 10% MeOD) δ_{H}: 8.74 (d, *J*=1.4Hz, 1H), 7.66 (d, *J*=7.5Hz, 2H), 7.44 - 7.50 (m, 2H), 7.30 (t, *J*=7.7Hz, 2H), 7.20 (t, *J*=7.4Hz, 2H), 6.97 - 7.02 (m, 1H), 4.38 (dd, *J*=10.7, 6.9Hz, 2H), 4.24 (dd, *J*=10.5, 6.7Hz, 1H), 4.04 (s, 3H), 3.75 - 3.79 (m, 1H), 3.15 - 3.24 (m, 1H), 3.04 (q, *J*=7.5Hz, 1H), 1.49 - 1.57 (m, 1 H), 1.37 - 1.44 (m, 1H) ,1.24 - 1.31 (m, 7H), 0.89 - 1.01 (m, 2H). MS (ES⁺) 642.8 (100%, [M+Na]⁺).

### (21): (R)-6-Hydroxy-7-((R)-1-{(R)-1-[1-(methoxycarbonylmethyl-carbamoyl)-cyclopropylcarbamoyl]-ethylcarbamoyl}-2-thiazol-4-yl-ethylcarbamoyl)-heptanoic acid tert-butyl ester

To a solution of **20** (650mg, 1.07mmol, 1 eq) in MeCN (22mL) was added Et₂NH (2.2mL, 10%v/v) dropwise at rt under Ar(g). The solution was stirred at rt for 2h, then the solvent was removed *in vacuo.* The excess of amine was co-evaporated with MeCN (3 x 10mL), then with a 1:5 mixture of CH₂Cl₂/hexane (10mL). A white solid was obtained and the flask was dried under high vacuum for 2h. To a solution of (*R*)-3-Hydroxy-octanedioic acid 8-tert-butyl ester **9** (291mg, 1.18mmol, 1.1 eq) [prepared by Chempartner Ltd, Shanghai, China] in MeCN (20mL) at 0°C was added PyBOP (614mg, 1.18mmol, 1.1 eq) and N-ethyldiisopropylamine (468µL, 2.68mmol, 2.5eq) under Ar(g). The crude amine, dissolved in CH₂Cl₂ (20mL) was added in dropwise fashion, and the reaction mixture was left to warm to rt overnight. The mixture was then concentrated in *vacuo* and the residue was further purified by silica gel column chromatography with CH₂Cl₂/MeOH (19:1 → 9:1) to yield **21,** a white solid as an inseparable mixture of 2 diastereoisomers (2:1 ratio) (650mg, 97% overall).
¹H NMR [major diastereoisomer] (400 MHz, CDCl₃) δ_{H}: 8.82 (d, *J*=1.9Hz, 1H), 7.79 (s, 1 H), 7.67 (d, *J*=6.4Hz, 1 H), 7.35 - 7.41 (m, 1 H), 7.22 (d, *J*=1.9Hz, 1 H), 4.70 (q, *J*=5.6Hz, 1H), 4.16 - 4.27 (m, 2H), 4.04 (dd, *J*=11.8, 5.6Hz, 3H), 3.71 (s, 3H), 3.35 - 3.44 (m, 2H), 2.42 (dd, *J*=13.6, 2.9Hz, 1H), 2.27 - 2.32 (m, 1H), 2.19 - 2.26 (m, 3H), 1.53 - 1.59 (m, 4H), 1.46 - 1.49 (m, 3H), 1.44 (s, 9H), 1.26 - 1.29 (m, 2H), 1.24 (d, *J*=7.3Hz, 3H), 1.00 - 1.13 (m, 2H). MS (ES⁺) 648.8 (100%, [M+Na]⁺).

### Compound XII: 5-((6R,9R,13R)-6-Methyl-5,8,11,15,18-pentaoxo-9-thiazol-4-ylmethyl-14-oxa-4,7,10,17-tetraaza-spiro[2.15]octadec-13-yl)-pentanoic acid tert-butyl ester

To a solution of 21 (650mg, 1.04mmol, 1eq) in THF (35mL) at 0°C was added a solution of LiOH (37.3mg, 1.56mmol, 1.5eq) in H₂O (7mL) dropwise. The mixture was stirred for 2h, then quenched with 1N HCl (4mL) and brine (5mL). The organic layer was separated and the resulting aqueous layer was further extracted with EtOAc (2 x 10mL) and CH₂Cl₂ (10mL). The combined organic extracts were dried over MgSO₄ and the solvent was removed *in vacuo.* The resulting carboxylic acid was then dried under high vacuum for 2h. To a solution of MNBA (429mg, 1.25mmol, 1.2eq) and DMAP (305mg, 2.5mmol, 2.4eq) in CH₂Cl₂ (0.80 L) was added a solution of the crude carboxylic acid in CH₂Cl₂ (320mL) and THF (20mL) dropwise over 3h. The reaction mixture was then left to stir at rt overnight. The solvent was then removed *in vacuo.* Purification by silica gel column chromatography with CH₂Cl₂/MeOH (16:1 → 9:1) yielded **compound XII** (187mg, 31%) as a pale yellow solid.
¹H NMR (400 MHz, CDCl₃) δ_{H}: 8.82 (d, *J*=1.6Hz, 2H), 7.97 (d, *J*=8.2Hz, 1 H), 7.68 (t, *J*=5.4Hz, 2H), 7.51 - 7.58 (m, 3H), 7.46 (t, *J*=7.9Hz, 1H), 5.21 - 5.29 (m, 1H), 4.72 - 4.82 (m, 1H), 4.14 (dd, *J*=16.4, 6.6Hz, 1 H), 4.02 - 4.07 (m, 1H), 3.93 (dd, *J*=16.3, 4.7Hz, 1H), 3.44 (dd, *J*=14.8, 5.6Hz, 1 H), 3.33 (dd, *J*=14.8, 6.7Hz, 1 H), 2.50 (s, 3H), 2.19 (t, *J*=7.4Hz, 2H), 1.50 - 1.60 (m, 4H), 1.43 (s, 9H), 1.31 - 1.37 (m, 5H), 0.95 - 1.13 (m, 2H). MS (ES⁺) 616.6 (100%, [M+Na]⁺).

### Compound XIII: 5-((6R,9R,13R)-6-Methyl-5,8,11,15,18-pentaoxo-9-thiazol-4-ylmethyl-14-oxa-4,7,10,17-tetraaza-spiro[2.15]octadec-13-yl)-pentanoic acid

To a solution of **compound XII** (187mg, 0.32mmol, 1eq) in TFA (2.35mL, 32mmol, 100eq) was added at 0 °C triethylsilane (151 µl, 0.95mmol, 3.0eq). The reaction mixture was stirred for 3h, then warmed up to rt. The solvent was removed *in vacuo.* Purification by silica gel column chromatography with CH₂Cl₂/MeOH (9:1 -> 6:1 + 0.1% AcOH) yielded **compound XIII** (150mg, 88%) as a white solid;
¹H NMR (400 MHz, CDCl₃ + 10% MeOD) δ_{H}: 8.75 (d, *J*=1.4Hz, 1H), 7.19 (d, *J*=1.3Hz, 1 H), 5.26 (s, 1 H), 4.60 (t, *J*=6.5Hz, 1 H), 4.08 (d, *J*=16.4Hz, 1 H), 3.85 (q, *J*=7.0Hz, 1H), 3.79 (d, *J*=16.3Hz, 1 H), 3.25 (dd, *J*=14.9, 5.6Hz, 1 H), 3.16 (dd, *J*=14.9, 7.7Hz, 1H), 2.45 (dd, *J*=14.6, 10.4Hz, 1H), 2.34 (dd, *J*=14.4, 2.3Hz, 1H), 2.23 (t, *J*=7.1Hz, 2H), 1.98 (s, 2H), 1.49 - 1.66 (m, 4H), 1.47 (d, *J*=2.1Hz, 2H), 1.26 - 1.32 (m, 1 H), 1.18 - 1.26 (m, 4H), 1.01 - 1.07 (m, 1 H), 0.90 - 0.96 (m, 1 H). MS (ES⁺) 537.8 (100%, [M+Na]⁺).

### Compound XIV: Methyl 5-((6R,9R,13R)-6-methyl-5,8,11,15,18-pentaoxo-9-(thiazol-4-ylmethyl)-14-oxa-4,7,10,17-tetraazaspiro[2,15]octadecan-13-yl)pentanoate (reference compound)

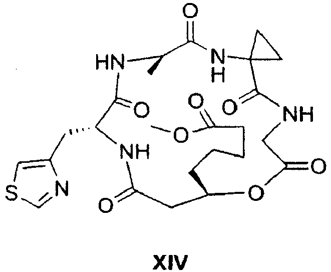

To a solution of **compound** XIII (19mg, 0.04mmol, 1eq) in THF (1mL) was added *ⁱ*BuOCOCl (9µL, 0.04mmol, 1eq) followed by Et₃N (10µL, 0.07mmol, 2eq) under Ar(g). After 1h, TMS-NHOH (4.8 µL, 0.04mmol, 1.1eq) was added and the reaction mixture was left to stir at rt overnight. The mixture was then quenched with MeOH (3mL) and left to stir for 2h before being concentrated *in vacuo* and purified by silica gel column chromatography with CH₂Cl₂/MeOH (19:1 -> 9:1), then by SCX-3 cartridge with CH₂Cl₂/MeOH (1:0 -> 0:1) and NH₃ in MeOH (0.1 M -> 0.3M) to yield **compound XIV** as a white solid (12mg, 62% yield).
¹H NMR (400 MHz, CDCl₃) δ_{H}: 8.80 (d, *J*=1.4H₂, 1H), 8.00 (d, *J*=5.5Hz, 1H), 7.59 - 7.67 (m, 2H), 7.48 (d, *J*=6.9Hz, 1 H), 7.18 - 7.22 (m, 1 H), 5.22 (q, *J*=6.0Hz, 1H), 4.69 (q, *J*=5.9Hz, 1H), 4.05 (t, *J*=5.2Hz, 3H), 3.67 (s, 3H), 3.45 (dd, *J*=14.9, 6.0Hz, 1 H), 3.31 (dd, *J*=14.9, 5.5Hz, 1 H), 2.56 (dd, *J*=14.3, 2.6Hz, 1 H), 2.47 (dd, *J*=14.2, 9.2Hz, 1H), 2.31 (t, *J*=7.2Hz, 2H), 1.59 - 1.66 (m, 3H), 1.48 - 1.58 (m, 3H), 1.29 - 1.33 (m, 4H), 1.21 - 1.28 (m, 1H), 0.96 - 1.11 (m, 2H). MS (ES⁺) 586.5 (100%, [M+Na]⁺).

### Compound XV: 5-((6R,9R,13R)-6-Methyl-5,8,11,15,18-pentaoxo-9-pyridin-3-ylmethyl-14-oxa-4,7,10,17-tetraaza-spiro[2.15]octadec-13-yl)-pentanoic acid tert-butyl ester (reference compound) and Compound XVI: 5-((6R,9R,13R)-6-Methyl-5,8,11,15,18-pentaoxo-9-pyridin-3-ylmethyl-14-oxa-4,7,10,17-tetraaza-spiro[2.15]octadec-13-yl)-pentanoic acid

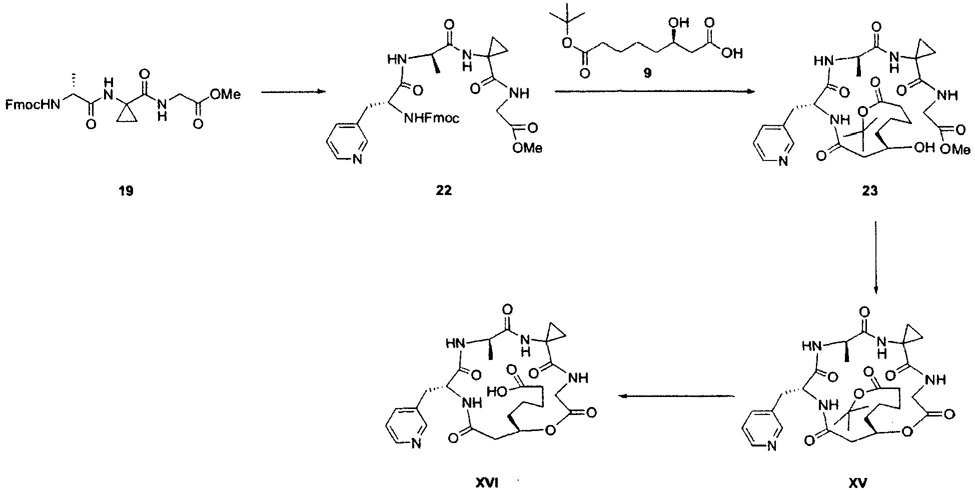

### (22): [(1-{(R)-2-[(R)-2-(9H-Fluoren-9-ylmethoxycarbonylamino)-3-pyridin-3-yl-propionylamino]-propionylamino}-cyclopropanecarbonyl)-amino]-acetic acid methyl ester

To a solution of ({1-[(*R*)-2-(9H-Fluoren-9-ylmethoxycarbonylamino)-propionylamino]-cyclopropanecarbonyl}-amino)-acetic acid methyl ester, **19** (600mg, 1.33mmol, 1eq (prepared by Chempartner Ltd, Shanghai, China)) in MeCN (27mL) was added Et₂NH (2.7mL, 10%v/v) dropwise at rt under Ar(g). The solution was stirred at rt for 2h, then the solvent was removed in *vacuo.* The excess of amine was co-evaporated with MeCN (3 x 10mL), then with a 1:5 mixture of CH₂Cl₂/hexane (10mL). A white solid was obtained and the flask was dried under high vacuum for 2h. To a solution of Fmoc-D-3-PyridylAla (568mg, 1.46mmol, 1.1eq) in MeCN (20mL) at 0°C was added 760mg of PyBOP (1.46mmol, 1.1 eq) and N-ethyldiisopropylamine (580µL, 3.32mmol, 2.5eq) under Ar(g). The crude amine, dissolved in CH₂Cl₂ (20mL) was added to the mixture dropwise. The reaction mixture was then left to warm to rt overnight. The mixture was then concentrated in *vacuo* and the residue was further purified by silica gel column chromatography with CH₂Cl₂/MeOH (19:1 → 9:1) to yield **22** as a white solid (677mg, 83%).
¹H NMR (400 MHz, CDCl₃) δ_{H}: 8.46 (d, *J*=4.7Hz, 2H), 7.76 (d, *J*=7.5Hz, 2H), 7.52 (d, *J*=7.5Hz, 2H), 7.36 - 7.45 (m, 3H), 7.28 - 7.34 (m, 2H), 7.21 - 7.26 (m, 1 H), 6.99 - 7.15 (m, 2H), 5.70 - 5.85 (m, 1 H), 4.30 - 4.51 (m, 3H), 4.08 - 4.20 (m, 2H), 3.97 - 4.07 (m, 1 H), 3.69 (s, 3H), 1.57 - 1.64 (m, 1 H), 1.51 - 1.57 (m, 1 H), 1.39 - 1.49 (m, 2H), 1.29 - 1.37 (m, 5H), 0.94 - 1.05 (m, 2H). MS (ES⁺) 636.2 (100%, [M+Na]⁺).

### (23): (R)-6-Hydroxy-7-((R)-1-{(R)-1-[1-(methoxycarbonylmethyl-carbamoyl)-cyclopropylcarbamoyl]-ethylcarbamoyl}-2-pyridin-3-yl-ethylcarbamoyl)-heptanoic acid tert-butyl ester

To a solution of 22 (677mg, 1.10mmol, 1eq) in MeCN (22mL) was added Et₂NH (2.2mL, 10%v/v) dropwise at rt under Ar(g). The solution was stirred at rt for 2h, then the solvent was removed in *vacuo.* The excess of amine was co-evaporated with MeCN (3 x 10mL), then with a 1:5 mixture of CH₂Cl₂/hexane (10mL). A white solid was obtained and the flask was dried under high vacuum for 2h. To a solution of (*R*)-3-Hydroxy-octanedioic acid 8-tert-butyl ester **9** (270mg, 1.21 mmol, 1.1 eq (prepared by Chempartner Ltd, Shanghai, China)) n MeCN (18mL) at 0°C was added PyBOP (629mg, 1.21mmol, 1.1eq) and of N-ethyldiisopropylamine (479µL, 2.75mmol, 2.5eq) under Ar(g). The crude amine, dissolved in CH₂Cl₂ (18mL) was added to the mixture dropwise. The reaction mixture was left to warm to rt overnight. The mixture was then concentrated in *vacuo* and the residue was further purified by silica gel column chromatography with CH₂Cl₂/MeOH (19:1 -> 6:1) to yield **23** as a white solid (753mg, 91%).
¹H NMR (400 MHz, CDCl₃) δ_{H}: 8.53 - 8.56 (m, 1H), 8.44 - 8.47 (m, 1H), 7.67 - 7.72 (m, 2H), 7.56 - 7.60 (m, 1 H), 7.43 - 7.49 (m, 1 H), 7.37 - 7.42 (m, 1 H), 7.31 - 7.35 (m, 1 H), 4.61 - 4.71 (m, 1H), 4.06 - 4.14 (m, 2H), 3.93 - 4.01 (m, 3H), 3.71 (s, 3H), 3.20 - 3.29 (m, 1 H), 3.05 - 3.14 (m, 1 H), 2.33 - 2.40 (m, 1 H), 2.26 - 2.32 (m, 1 H), 2.17 - 2.23 (m, 3H), 1.51 - 1.57 (m, 3H), 1.43 (s, 9H), 1.30 - 1.34 (m, 5H), 1.01 - 1.06 (m, 2H). MS (ES⁺) 642.8 (100%, [M+Na]⁺).

### Compound XV: 5-((6R,9R,13R)-6-Methyl-5,8,11,15,18-pentaoxo-9-pyridin-3-ylmethyl-14-oxa-4,7,10,17-tetraaza-spiro[2.15]octadec-13-yl)-pentanoic acid tert-butyl ester

To a solution of 23 (753mg, 1.21 mmol, 1eq) in THF (41 mL) at 0 °C was added a solution of LiOH (44mg, 1.82mmol, 1.5eq) in H₂O (8mL) dropwise. The mixture was stirred for 2h, then quenched with 1N HCl (8mL) and brine (5mL). The organic layer was separated and the resulting aqueous layer was further extracted with EtOAc (2 x 10mL) and CH₂Cl₂ (10mL). The combined organic extracts were dried over MgSO₄ and the solvent was removed *in vacuo.* The resulting carboxylic acid was then dried under high vacuum for 2h. To a solution of MNBA (500mg, 1.45mmol, 1.2eq) and DMAP (354mg, 2.9mmol, 2.4eq) in CH₂Cl₂ (0.88 L) was added a solution of the crude carboxylic acid in CH₂Cl₂ (400mL) and THF (40mL) dropwise over 3h. The reaction mixture was then left to stir at rt overnight. The solvent was removed *in vacuo.* Purification by silica gel column chromatography with CH₂Cl₂/MeOH (9:1 → 4:1) yielded **compound XV** (228mg, 32%) as a pale yellow solid.
¹H NMR (400 MHz, CDCl₃ + 10% MeOD) δ_{H}: 7.92 (m, 1H), 7.79 (m, 2H), 7.70 (d, *J*=7.7Hz, 1H), 6.61 (d, *J*=7.7Hz, 2H), 5.05 - 5.14 (m, 1H), 4.20 - 4.25 (m, 1H), 4.07 (m, 1 H), 3.86 (q, *J*=7.1Hz, 1 H), 3.38 - 3.45 (m, 2H), 3.05 (m, 1 H), 2.86 (m, 1 H), 2.20 (m, 1 H), 1.93 - 2.06 (m, 4H), 1.64 (m, 2H), 1.43 (s, 9H), 1.17 - 1.26 (m, 2H), 1.10 (m, 6H), 0.90 - 1.05 (m, 2H). MS (ES⁺) 610.7 (100%, [M+Na]⁺).

### Compound XVI: 5-((6R,9R,13R)-6-Methyl-5,8,11,15,18-pentaoxo-9-pyridin-3-ylmethyl-14-oxa-4,7,10,17-tetraaza-spiro[2.15]octadec-13-yl)-pentanoic acid

To a solution of **compound XV** (228mg, 0.39mmol, 1eq) in TFA (3.0mL, 39mmol, 100eq) was added at 0°C triethylsilane (185 µL, 1.16mmol, 3.0eq). The reaction mixture was stirred for 3h, then warmed to rt. The solvent was removed *in vacuo.* Purification by silica gel column chromatography with CH₂Cl₂/MeOH (6:1 -> 3:1 + 0.1% AcOH) yielded **compound XVI** (117mg, 57%) as a white solid.
¹H NMR (400 MHz, CDCl₃ + 10% MeOD) δ_{H}: 7.86 (dd, *J*=8.0, 3.7Hz, 1H), 7.79 (d, *J*=7.6Hz, 2H), 7.68 (d, *J*=7.7Hz, 1H), 6.61 (d, *J*=7.7Hz, 2H), 5.03 - 5.13 (m, 1H), 4.23 - 4.28 (m, 1H), 4.01 (dd, *J*=16.5, 8.3Hz, 1H), 3.83 (q, *J*=7.2Hz, 1 H), 3.42 - 3.49 (m, 2H), 2.84 - 2.92 (m, 1 H), 2.71 - 2.80 (m, 1 H), 2.17 - 2.27 (m, 1H), 1.95 - 2.05 (m, 4H), 1.74 (d, *J*=6.8Hz, 3H), 1.07 - 1.15 (m, 2H), 1.04 (d, *J*=7.2Hz, 6H), 0.85 - 0.93 (m, 2H). MS (ES⁺) 554.8 (100%, [M+Na]⁺).

## Claims

1. A compound of Structure III or IV: 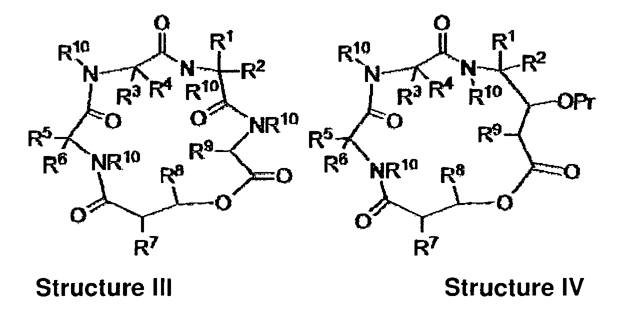 or a pharmaceutically acceptable salt thereof,
wherein:
R₁, R₂, R₃, R₄, R₅, R₆, R₇, and R₉ are the same or different and represent hydrogen or natural or unnatural amino acid side-chain moiety or R₁ and R₂, R₃ and R₄, or R₅ and R₆, taken together with the carbon atom to which they are attached, from a cyclic moiety containing between 3 and 8 carbon atoms;
each R₁₀ is the same of different and represents hydrogen or C₁-C₆ alkyl, C₂-C₆ alkenyl or C₂-C₆ alkynyl;
Pr is hydrogen or an alcohol protecting group;
R₈ represents -CR₁₁R₁₁-CR₁₁R₁₁-(CH₂)ₙ-R₁₂, CR₁₁=CR₁₁-(CH₂)ₙ-R₁₂, or -C≡C-(CH₂)ₙ-R₁₂;
R₁₁ is hydrogen, C₁-C₆ alkyl, aryl, C₂-C₆ alkenyl, C₂-C₆ alkynyl or heteroaryl;
R₁₂ is a linear or cyclic metal-chelating moiety, capable of binding with zinc in the active site of histone deacetylase, and is represented by a structure shown below: 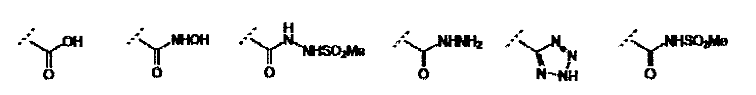 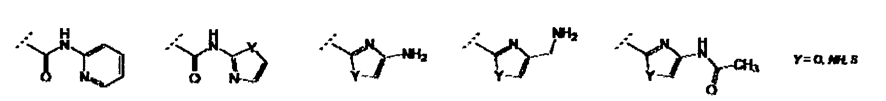 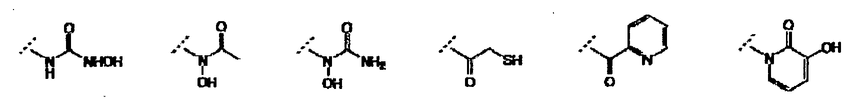 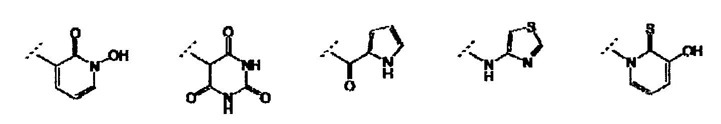 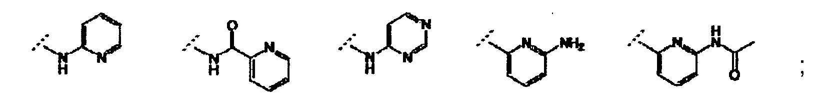 and
n is an integer from 0 to 7,
each unnatural amino acid side-chain moiety is independently selected from -(CH₂)₂-C(O)-O-C(CH₃)₃ (glutamic acid *t*-butyl ester), -(CH₂)₄-NH-C(O)-O-C(CH₃)₃ (N_{ε}-(*tert*-butoxycarbonyl)-lysine), -(CH₂)₃-NH-C(O)NH₂ (citrulline), -CH₂-CH₂OH (homoserine), -(CH₂)₂-CH₂NH₂ (ornithine), C₁-C₆ alkyl, C₂-C₆ alkenyl, C₂-C₆ alkynyl, aryl or a saturated or unsaturated heterocycle. -(CR₁₁R₁₁)ₓ-NR₁₁C(O)NR₁₁R₁₁, -(CR₁₁R₁₁)ₓ-NR₁₁C(O)NR₁₁R₁₃, -(CR₁₁R₁₁)ₓ-NR₁₁C(O)OR₁₄, -(CR₁₁R₁₁)ₓ-NR₁₁C(O)R₉, -(CR₁₁R₁₁)ₓ-NR₁₁C(O)R₁₃, -(CR₁₁R₁₁)ₓ-NR₁₁SO₂NR₁₁R₁₁, -(CR₁₁R₁₁)ₓ-NR₁₁SO₂NR₁₁R₁₃, -(CR₁₁R₁₁)ₓ-NR₁₁SO₃R₁₄, -(CR₁₁R₁₁)ₓ-NR₁₁SO₂R₁₄, -(CR₁₁R₁₁)ₓ-NR₁₁SO₂R₁₃, -(CR₁₁R₁₁)ₓ-C(O)NR₁₁R₁₁, -(CR₁₁R₁₁)ₓ-C(O)NR₁₁R₁₃, -(CR₁₁R₁₁)ₓ-CO₂R₁₁, -(CR₁₁R₁₁)ₓ-C(O)R₁₃, -(CR₁₁R₁₁)ₓ-SO₂NR₁₁R₁₁, -(CR₁₁R₁₁)ₓ-SO₂NR₁₁R₁₃, -(CR₁₁R₁₁)ₓ-SO₂R₁₃, -(CR₁₁R₁₁)ₓ-Ar;
x is an integer from 1 to 10;
R₁₃ is NR₁₁-C(O)R₁₄ or NR₁₁-SO₂R₁₄;
R₁₄ is C₁-C₆ alkyl, aryl, C₂-C₆ alkenyl, C₂-C₆ alkynyl or heteroaryl; and
Ar is functionalized and unfunctionalized phenyl, and functionalized and unfunctionalized 5- and 6-membered unsaturated aryl and heteroaryl rings.

2. A compound according to claim 1, wherein R₈ is CR₁₁R₁₁-CR₁₁R₁₁-(CH₂)ₙ-R₁₂, and wherein R₁₂ is an ester, a carboxylic acid or -C(=O)NH-OH and R₁₁ is as defined in claim 1.

3. A compound according to any preceding claim, wherein the natural or unnatural amino acid side chain moiety is -CH₃ (Alanine), -CH(CH₃)₂ (Valine), -CH₂CH(CH₃)₂ (Leucine), -CH(CH₃)CH₂CH₃ (Isoleucine), -(CH₂)₄NH₂ (Lysine), -(CH₂)₃NHC(=NH)NH₂ (Arginine), -CH₂-(5-1*H*-imidazolyl) (Histidine), -CH₂CONH₂ (Asparagine), -CH₂CH₂CONH₂ (Glutamine), -CH₂COOH (Aspartic acid), -CH₂CH₂COOH (Glutamic acid), -CH₂-phenyl (Phenylalanine), -CH₂-(4-OH-phenyl) (Tyrosine), -CH₂-(3-1*H*-indolyl) (Tryptophan), -CH₂SH (Cysteine), -CH₂CH₂SCH₃ (Methionine), -CH₂OH (Serine), and -CH(OH)CH₃ (Threonine), -(CH₂)₂-C(O)-O-C(CH₃)₃ (glutamic acid *t*-butyl ester), -(CH₂)₄-NH-C(O)-O-C(CH₃)₃ (N_{ε}-(*tert*-butoxycarbonyl)-lysine), -(CH₂)₃-NH-C(O)NH₂ (citrulline), -CH₂-CH₂OH (homoserine) and -(CH₂)₃NH₂ (ornithine), -H(Glycine), C₁-C₆ alkyl, C₂-C₆ alkenyl, C₂-C₆ alkynyl, aryl or a saturated or unsaturated heterocycle which can be functionalized or unfunctionalized.

4. A compound according to any preceding claim, which has one of the Structures shown below:

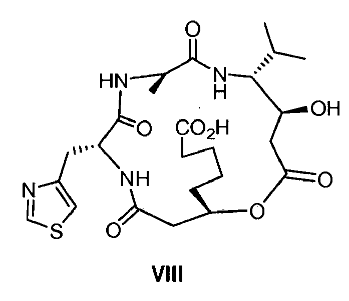

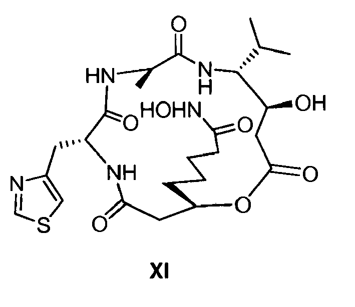

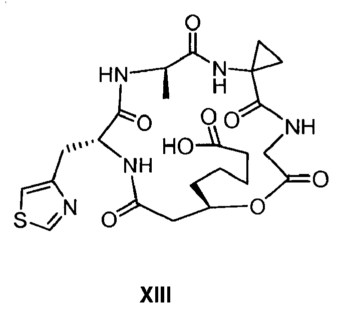

5. A compound according to any preceding claim, for use in therapy.

6. A compound for use according to claim 5, wherein the condition is cancer, cardiac hypertrophy, chronic heart failure, an inflammatory condition, a cardiovascular disease, a haemoglobinopathy, a thalassemia, a sickle cell disease, a CNS disorder, an autoimmune disease, diabetes, osteoporosis, MDS, benign prostatic hyperplasia, endometriosis, oral leukoplakia, a genentically related metabolic disorder, an infection, Rubens-Taybi, fragile X syndrome, alpha-1 antitrypsin deficiency, chronic lymphocytic leukaemia, breast cancer, prostate cancer, ovarian cancer, mesothelioma, T-cell lymphoma, cardiac hypertrophy, chronic heart failure or a skin inflammatory condition, in particular psoriasis, acne or eczema.

7. A compound according to any of claims 1 to 4, for use in accelerating wound healing, protecting hair follicles, or as an immunosuppressant.

8. A pharmaceutical composition comprising a compound according to any of claims 1 to 4 and a pharmaceutically acceptable carrier or diluent.

9. A composition according to claim 8, which is in a form suitable for oral, rectal, parenteral, intranasal or transdermal administration or administration by inhalation or by suppository.

10. A composition according to claim 9, which is in the form of granules, a tablet, capsule, troche, lozenge, aqueous or oily suspension, or dispersible powder.

11. A product containing (a) a compound according to any of claims 1 to 4, and (b) another inhibitor of HDAC, for simultaneous, separate or sequential use in therapy.

12. A product according to claim 11, wherein the therapy is of a condition listed in claim 6 or claim 7.

13. A product containing (a) a compound according to any of claims 1 to 4, and (b) a chemotherapeutic or antineoplastic agent, for simultaneous, separate or sequential use in therapy.

14. A product according to claim 13, wherein the therapy is of cancer.

## Patentansprüche

1. Eine Verbindung der Struktur III oder IV: 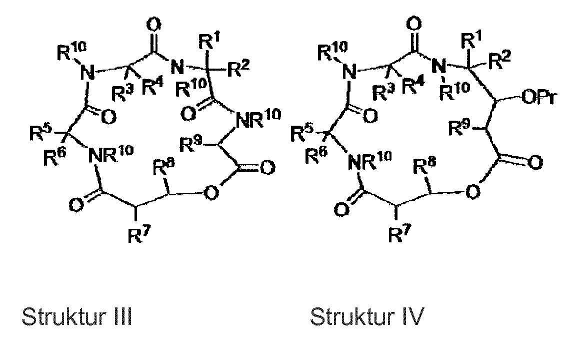 oder ein pharmazeutisch akzeptables Salz davon,
wobei:
R₁, R₂, R₃, R₄, R₅, R₆, R₇ und R₉ gleich oder verschieden sind und Wasserstoff oder einen natürlichen oder unnatürlichen Aminosäuren- Seitenkettenanteil repräsentieren, oder R₁ und R₂, R₃ und R₄, oder R₅ und R₆, zusammengenommen mit dem Kohlenstoffatom, an dem sie hängen, einen cyclischen Anteil bilden, der zwischen 3 und 8 Kohlenstoffatome enthält;
jedes R₁₀ gleich oder verschieden ist und Wasserstoff oder C₁-C₆-Alkyl, C₂-C₆-Alkenyl oder C₂-C₆-Alkinyl repräsentiert;
Pr Wasserstoff oder eine Alkohol-Schutzgruppe ist;
R₈ -CR₁₁R₁₁-CR₁₁R₁₁-(CH₂)ₙ-R₁₂, CR₁₁-CR₁₁-(CH₂)ₙ-R₁₂ oder -C≡C-(CH₂)ₙ-R₁₂ repräsentiert;
R₁₁ Wasserstoff, C₁-C₆-Alkyl, Aryl, C₂-C₆-Alkenyl, C₂-C₆-Alkinyl oder Heteroaryl ist;
R₁₂ ein linearer oder cyclischer metallchelatbildender Anteil ist, der zur Bindung mit Zink im aktiven Zentrum von Histondeacetylase fähig ist und durch eine unten gezeigte Struktur repräsentiert wird: 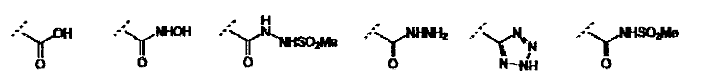 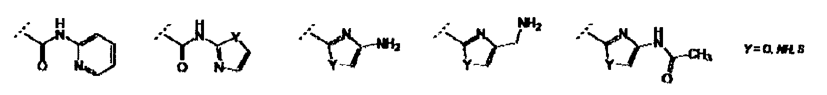 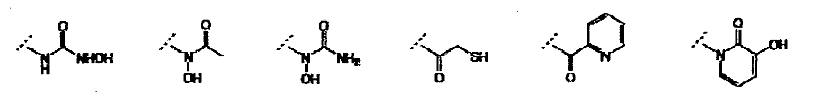 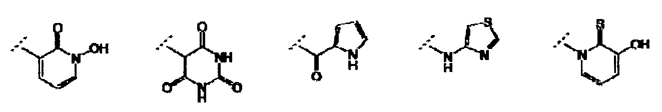 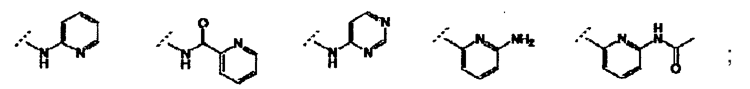 und
n eine ganze Zahl von 0 bis 7 ist,
jeder unnatürliche Aminosäuren-Seitenkettenanteil unabhängig ausgewählt ist aus -(CH₂)₂-C(O)-O-C(CH₃)₃ (Glutaminsäure-*t*-butylester), -(CH₂)₄-NH-C(O)-O-C(CH₃)₃ (N_{ε}-(*tert*-Butoxycarbonyl)-lysin), -(CH₂)₃-NH-C(O)NH₂ (Citrullin), -CH₂-CH₂OH (Homoserin), -(CH₂)₂-CH₂NH₂ (Ornithin), C₁-C₆-Alkyl, C₂-C₆-Alkenyl, C₂-C₆-Alkinyl, Aryl oder ein gesättigter oder ungesättigter Heterocyclus. -(CR₁₁R₁₁)ₓ-NR₁₁C(O)NR₁₁R₁₁, - (CR₁₁R₁₁)ₓ-NR₁₁C(O)NR₁₁R₁₃, -(CR₁₁R₁₁)ₓ-NR₁₁C(O)OR₁₄, -(CR₁₁R₁₁)ₓ-NR₁₁C(O)R₉, -(CR₁₁R₁₁)ₓ-NR₁₁C(O)R₁₃, -(CR₁₁R₁₁)ₓ-NR₁₁SO₂NR₁₁R₁₁, -(CR₁₁R₁₁)ₓ-NR₁₁SO₂NR₁₁R₁₃, - (CR₁₁R₁₁)ₓ-NR₁₁SO₃R₁₄, -(CR₁₁R₁₁)ₓ-NR₁₁SO₂R₁₄, -(CR₁₁R₁₁)ₓ-NR₁₁SO₂R₁₃, -(CR₁₁R₁₁)ₓ-C(O)NR₁₁R₁₁, -(CR₁₁R₁₁)ₓ-C(O)NR₁₁R₁₃, -(CR₁₁R₁₁)ₓ-CO₂R₁₁, -(CR₁₁R₁₁)ₓ-C(O)R₁₃, - (CR₁₁R₁₁)ₓ-SO₂NR₁₁R₁₁, -(CR₁₁R₁₁)ₓ-SO₂NR₁₁R₁₃, -(CR₁₁R₁₁)ₓ-SO₂R₁₃, -(CR₁₁R₁₁)ₓ-Ar,
x eine ganze Zahl von 1 bis 10 ist;
R₁₃ NR₁₁-C(O)R₁₄ oder NR₁₁-SO₂R₁₄ ist;
R₁₄ C₁-C₆-Alkyl, Aryl, C₂-C₆-Alkenyl, C₂-C₆-Alkinyl oder Heteroaryl ist; und
Ar funktionalisiertes und unfunktionalisiertes Phenyl, und funktionalisierte und unfunktionalisierte 5- und 6-gliedrige ungesättigte Aryl- und Heteroarylringe ist.

2. Eine Verbindung gemäß Anspruch 1, wobei R₈ CR₁₁R₁₁-CR₁₁R₁₁-(CH₂)ₙ- R₁₂ ist und wobei R₁₂ ein Ester, eine Carbonsäure oder -C(=O)NH-OH ist und R₁₁ wie in Anspruch 1 definiert ist.

3. Eine Verbindung gemäß einem vorhergehenden Anspruch, wobei der natürliche oder unnatürliche Aminosäuren-Seitenkettenanteil -CH₃ (Alanin), -CH(CH₃)₂ (Valin),-CH₂CH(CH₃)₂ (Leucin), -CH(CH₃)CH₂CH₃ (Isoleucin), -(CH₂)₄NH₂ (Lysin),-(CH₂)₃NHC(=NH)NH₂ (Arginin), -CH₂-(5-1*H*-Imidazolyl) (Histidin), -CH₂CONH₂ (Asparagin), -CH₂CH₂CONH₂ (Glutamin),
-CH₂COOH (Asparaginsäure), -CH₂CH₂COOH (Glutaminsäure), -CH₂-Phenyl (Phenylalanin), -CH₂-(4-OH-Phenyl) (Tyrosin), -CH₂-(3-1*H*-Indolyl) (Tryptophan), - CH₂SH (Cystein), -CH₂CH₂SCH₃ (Methionin), -CH₂OH (Serin) und -CH(OH)CH₃ (Threonin), -(CH₂)₂-C(O)-O-C(CH₃)₃ (Glutaminsäure-*t*-butylester), -(CH₂)₄-NH-C(O)-O-C(CH₃)₃ (N_{ε}-(*tert*-Butoxycarbonyl)-lysin), -(CH₂)₃-NH-C(O)NH₂ (Citrullin), -CH₂-CH₂OH (Homoserin), -(CH₂)₂-CH₂NH₂ (Ornithin), -H(Glycin), C₁-C₆-Alkyl, C₂-C₆-Alkenyl, C₂-C₆-Alkinyl, Aryl oder ein gesättigter oder ungesättigter Heterocyclus ist, der funktionalisiert oder unfunktionalisiert sein kann.

4. Eine Verbindung gemäß einem vorhergehenden Anspruch, die eine der unten gezeigten Strukturen aufweist:

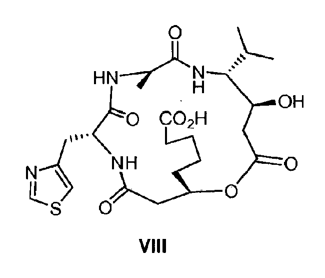

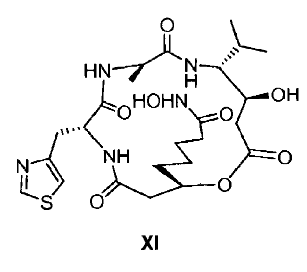

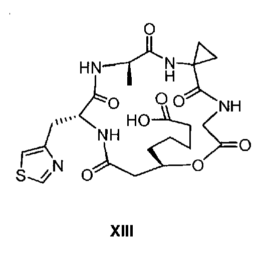

5. Eine Verbindung gemäß einem vorhergehenden Anspruch zur Verwendung in der Therapie.

6. Eine Verbindung zur Verwendung gemäß Anspruch 5, wobei die Erkrankung Krebs, Herzhypertrophie, chronische Herzinsuffizienz, eine entzündliche Erkrankung, eine kardiovaskuläre Krankheit, eine Hämoglobinopathie, eine Thalassämie, eine Sichelzellkrankheit, eine ZNS-Erkrankung, eine Autoimmunkrankheit, Diabetes, Osteoporose, MDS, benigne Prostatahyperplasie, Endometriose, orale Leukoplakie, eine genetisch bedingte Stoffwechselstörung, eine Infektion, Rubinstein-Taybi-Syndrom, Fragiles-X-Syndrom, Alpha-1-Antitrypsinmangel, chronische lymphatische Leukämie, Brustkrebs, Prostatakrebs, Eierstockkrebs, Mesotheliom, T-Zell-Lymphom, Herzhypertrophie, chronische Herzinsuffizienz oder eine entzündliche Hauterkrankung, insbesondere Psoriasis, Akne oder Ekzem, ist.

7. Eine Verbindung gemäß einem der Ansprüche 1 bis 4, zur Verwendung bei der Beschleunigung der Wundheilung, beim Schutz von Haarfollikeln oder als Immunsuppressivum.

8. Eine pharmazeutische Zusammensetzung, die eine Verbindung gemäß einem der Ansprüche 1 bis 4 und einen pharmazeutisch akzeptablen Träger oder ein Verdünnungsmittel beinhaltet.

9. Eine Zusammensetzung gemäß Anspruch 8, die in einer für die orale, rektale, parenterale, intranasale oder transdermale Verabreichung oder Verabreichung mittels Inhalation oder Zäpfchen geeigneten Form vorliegt.

10. Eine Zusammensetzung gemäß Anspruch 9, die in Form von Granulae, einer Tablette, Kapsel, Pastille, Lutschtablette, wässrigen oder öligen Suspension oder eines dispergierbaren Pulvers vorliegt.

11. Ein Produkt, das (a) eine Verbindung gemäß einem der Ansprüche 1 bis 4 und (b) einen anderen Hemmer von HDAC enthält, zur gleichzeitigen, separaten oder aufeinanderfolgenden Verwendung in der Therapie.

12. Ein Produkt gemäß Anspruch 11, wobei die Therapie für eine in Anspruch 6 oder Anspruch 7 aufgeführte Erkrankung ist.

13. Ein Produkt, das (a) eine Verbindung gemäß einem der Ansprüche 1 bis 4 und (b) ein chemotherapeutisches oder antineoplastisches Mittel enthält, zur gleichzeitigen, separaten oder aufeinanderfolgenden Verwendung in der Therapie.

14. Ein Produkt gemäß Anspruch 13, wobei die Therapie für Krebs ist.

## Revendications

1. Composé de structure III ou IV : 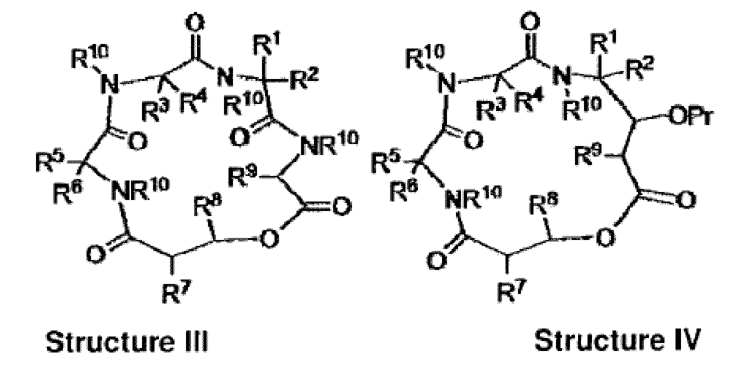 ou un sel acceptable sur le plan pharmaceutique de ce composé,
dans lequel :
R₁, R₂, R₃, R₄, R₅, R₆, R₇ et R₉ sont identiques ou différents et représentent un atome d'hydrogène ou une fraction de chaîne latérale d'un acide aminé naturel ou non naturel ou R₁ et R₂, R₃ et R₄ ou R₅ et R₆, ensemble avec l'atome de carbone auquel ils sont reliés, forment une fraction cyclique contenant entre 3 et 8 atomes de carbone ;
chaque R₁₀ est identique ou différent et représente un atome d'hydrogène ou un alkyle en C₁ à C₆, un alkényle en C₂ à C₆ ou un alkynyle en C₂ à C₆ ;
Pr représente un atome d'hydrogène ou un groupe protecteur d'alcool ;
R₈ représente -CR₁₁R₁₁-CR₁₁R₁₁-(CH₂)ₙ-R₁₂, CR₁₁=CR₁₁-(CH₂)ₙ-R₁₂ ou -C≡C-(CH₂)ₙ-R₁₂ ;
R₁₁ représente un atome d'hydrogène, un alkyle en C₁ à C₆, un aryle, un alkényle en C₂ à C₆, un alkynyle en C₂ à C₆ ou un hétéroaryle ;
R₁₂ est une fraction métallique chélatante linéaire ou cyclique capable de se lier au zinc dans le site actif de l'histone désacétylase et n est représenté par une structure illustrée ci-dessous : 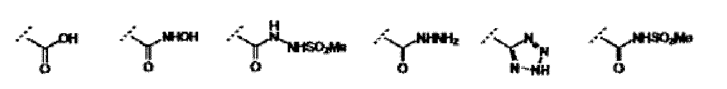 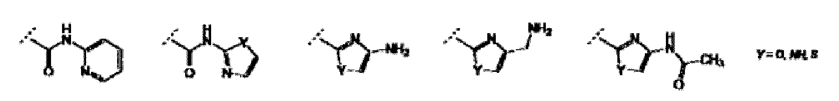 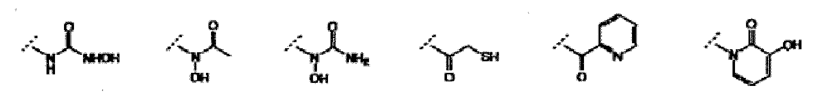 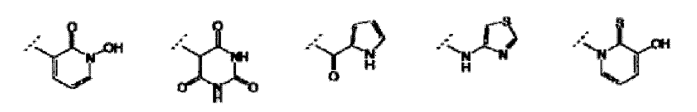 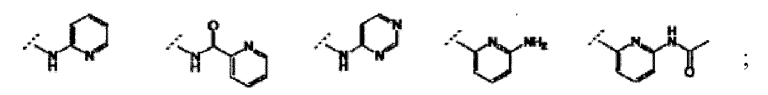 et
N représente un nombre entier valant 0 à 7,
chaque fraction de chaîne latérale d'un acide aminé non naturel est indépendamment sélectionnée parmi -(CH₂)₂-C(O)-O-C(CH₃)₃ (*tert*-butyle ester de l'acide glutamique), -(CH₂)₄-NH-C(O)-O-C(CH₃)₃ (N_{ε}-(*tert-*butoxycarbonyle)-lysine), -(CH₂)₃-NH-C(O)NH₂ (citrulline), -CH₂-CH₂OH (homosérine, -(CH₂)₂-CH₂NH₂ (ornithine), alkyle en C₁ à C₆, alkényle en C₂ à C₆, alkynyle en C₂ à C₆, aryle ou un hétérocycle saturé ou insaturé. - (CR₁₁R₁₁)ₓ-NR₁₁C(O)NR₁₁R₁₁, -(CR₁₁R₁₁)ₓ-NR₁₁C(O)NR₁₁R₁₃, - (CR₁₁R₁₁)ₓ-NR₁₁C(O)OR₁₄, -(CR₁₁R₁₁)ₓ-NR₁₁C(O)R₉, -(CR₁₁R₁₁)ₓ-NR₁₁C(O)R₁₃, -(CR₁₁R₁₁)ₓ-NR₁₁SO₂NR₁₁R₁₁, -(CR₁₁R₁₁)ₓ-NR₁₁SO₂NR₁₁R₁₃, -(CR₁₁R₁₁)ₓ-NR₁₁SO₃R₁₄, -(CR₁₁R₁₁)ₓ-NR₁₁SO₂R₁₄, - (CR₁₁R₁₁)ₓ-NR₁₁SO₂R₁₃, -(CR₁₁R₁₁)ₓ-C(O)NR₁₁R₁₁, -(CR₁₁R₁₁)ₓ-C(O)NR₁₁R₁₃, -(CR₁₁R₁₁)ₓ-CO₂R₁₁, -(CR₁₁R₁₁)ₓ-C(O)R₁₃, -(CR₁₁R₁₁)ₓ-SO₂NR₁₁R₁₁, -(CR₁₁R₁₁)ₓ-SO₂NR₁₁R₁₃, -(CR₁₁R₁₁)ₓ-SO₂R₁₃, -(CR₁₁R₁₁)ₓ-Ar;
x représente un entier valant de 1 à 10 ;
R₁₃ représente NR₁₁-C(O)R₁₄ ou NR₁₁-SO₂R₁₄,
R₁₄ représente un alkyle en C₁ à C₆, un aryle, un alkényle en C₂ à C₆, un alkynyle en C₂ à C₆ ou un hétéroaryle ; et
Ar représente un phényle fonctionnalisé et non fonctionnalisé et un aryle insaturé fonctionnalisé et non fonctionnalisé à 5 et 6 éléments.

2. Composé selon la revendication 1, dans lequel R₈ représente CR₁₁R₁₁-CR₁₁R₁₁-(CH₂)ₙ-R₁₂ et dans lequel R₁₂ représente un ester, un acide carboxylique ou -C(=O)NH-OH et R₁₁, représente les composés définis à la revendication 1.

3. Composé selon l'une quelconque des revendications précédentes, dans lequel la fraction de chaîne latérale de l'acide aminé naturel ou non naturel représente -CH₃ (Alanine), CH(CH₃)₂ (Valine) -CH₂CH(CH₃)₂ (Leucine), -CH(CH₃)CH₂CH₃ (Isoleucine), -(CH₂)₄NH₂ (Lysine),-(CH₂)₃NHC(=NH)NH₂ (Arginine), -CH₂-(5-1*H*-imidazolyle) (Histidine),-CH₂CONH₂ (Asparagine), -CH₂CH₂CONH₂ (Glutamine), -CH₂COOH (Acide aspartique), -CH₂CH₂COOH (acide glutamique), -CH₂-phényle (Phénylalanine), -CH₂-(4-OH-phényle) (Tyrosine), -CH₂-(3-1*H*-indolyle) (Tryptophane), -CH₂SH (Cystéine), -CH₂CH₂SCH₃ (Méthionine), -CH₂OH (Sérine) et -CH(OH)CH₃ (Thréonine), -(CH₂)₂-C(O)-O-C(CH₃)₃ (*tert-*butyle ester de l'acide glutamique), -(CH₂)₄-NH-C(O)-O-C(CH₃)₃, (N_{ε}-(*tert*-butoxycarbonyle)-lysine, -(CH₂)₃-NH-C(O)NH₂ (citrulline, -CH₂-CH₂OH (homosérine) et -(CH₂)₃NH₂ (ornithine), H(Glycine), alkyle en C₁ à C₆, alkényle en C₂ à C₆, alkynyle en C₂ à C₆, aryle ou un hétérocycle saturé ou insaturé qui peut être fonctionnalisé ou non fonctionnalisé.

4. Composé selon l'une quelconque des revendications précédentes, qui possède l'une des structures illustrées ci-dessous :

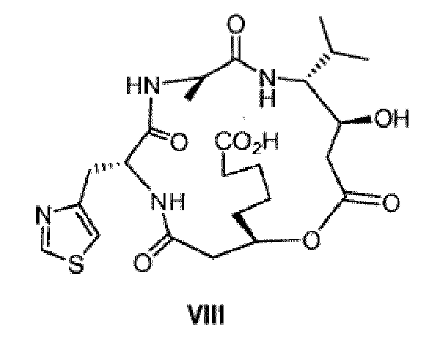

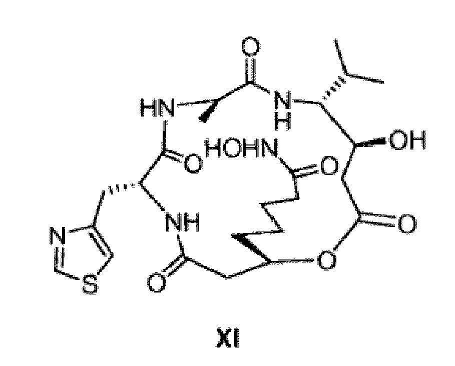

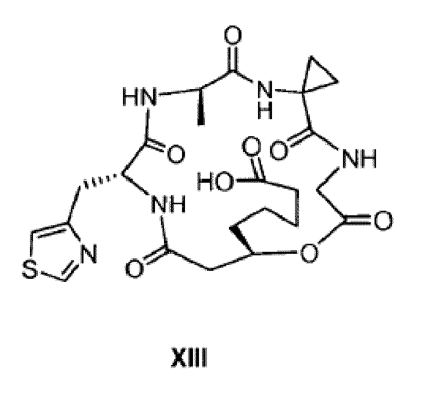

5. Composé selon l'une des revendications précédentes, destiné à une utilisation en thérapie.

6. Composé pour utilisation selon la revendication 5, dans lequel la pathologie est le cancer, l'hypertrophie cardiaque, la défaillance cardiaque chronique, une condition inflammatoire, une maladie cardiovasculaire, une hémoglobinopathie, une thalassémie, une drépanocytose, une maladie du système nerveux central (SNC), une maladie auto-immune, un diabète, une ostéoporose, un syndrome myélodysplasique (MDS), une hyperplasie prostatique bénigne, une endométriose, une leucoplasie orale, un trouble métabolique génétiquement associé, une infection, le syndrome de Rubinstein-Tabi, le syndrome X fragile, une carence en alpha-1 antitrypsine, une leucémie lymphocytique chronique, un cancer du sein, un cancer de la prostate, un cancer de l'ovaire, un mésothéliome, un lymphome des lymphocytes T, une hypertrophie cardiaque, une insuffisance cardiaque chronique ou un état cutané inflammatoire, en particulier le psoriasis, l'acné ou l'eczéma.

7. Composé selon l'une quelconque des revendications 1 à 4 pour utilisation dans l'accélération de la guérison de blessures, la protection des follicules pileux ou comme immunosuppresseur.

8. Composition pharmaceutique contenant un composé selon l'une quelconque des revendications 1 à 4 et un vecteur ou diluant acceptable du point de vue pharmaceutique.

9. Composition selon la revendication 8, qui se présente sous une forme appropriée à une administration par voie orale, rectale, parentérale, intranasale ou transdermique ou à une administration par inhalation ou par suppositoire.

10. Composition selon la revendication 9, qui se présente sous forme de granules, de comprimé, de capsule, de pastille, de losange, de suspension aqueuse ou huileuse, ou d'une poudre dispersible.

11. Produit contenant (a) un composé selon l'une quelconque des revendications 1 à 4, et (b) un autre inhibiteur de la HDAC (histone-désacétylase), destiné à une utilisation simultanée, séparée ou séquentielle sur le plan thérapeutique.

12. Produit selon la revendication 11, dans lequel la thérapie est une pathologie listée dans la revendication 6 ou la revendication 7.

13. Produit contenant (a) un composé selon l'une quelconque des revendications 1 à 4, et (b) un agent chimiothérapeutique ou antinéoplasique, destiné à une utilisation simultanée, séparée ou séquentielle sur le plan thérapeutique.

14. Produit selon la revendication 13, la thérapie étant celle du cancer.
